(19) Europäisches Patentamt
European Patent Office
Office européen des brevets

(11) EP 2 769 998 B1

(12) **EUROPEAN PATENT SPECIFICATION**

(45) Date of publication and mention of the grant of the patent:
**03.08.2016 Bulletin 2016/31**

(21) Application number: **12842413.2**

(22) Date of filing: **15.10.2012**

(51) Int Cl.:
***C07D 211/46*** (2006.01) ***C07D 211/58*** (2006.01)
***C08F 2/48*** (2006.01) ***C08F 220/36*** (2006.01)

(86) International application number:
**PCT/JP2012/076643**

(87) International publication number:
**WO 2013/058218 (25.04.2013 Gazette 2013/17)**

(54) **(METH)ACRYLATE COMPOUND, AND PHOTOCHROMIC CURABLE COMPOSITION CONTAINING SAID (METH)ACRYLATE COMPOUND**

(METH)ACRYLATVERBINDUNG UND PHOTOCHROME HÄRTBARE ZUSAMMENSETZUNG MIT DIESER (METH)ACRYLATVERBINDUNG

COMPOSÉ DE (MÉTH)ACRYLATE, ET COMPOSITION PHOTOCHROMIQUE DURCISSABLE LE CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2011 JP 2011227745**

(43) Date of publication of application:
**27.08.2014 Bulletin 2014/35**

(73) Proprietor: **Tokuyama Corporation**
**Shunan-shi, Yamaguchi 745-8648 (JP)**

(72) Inventors:
- **TAKENAKA, Junji**
  **Shunan-shi**
  **Yamaguchi 745-8648 (JP)**
- **MOMODA, Junji**
  **Shunan-shi**
  **Yamaguchi 745-8648 (JP)**

(74) Representative: **Duckworth, Timothy John**
**J A Kemp**
**14 South Square**
**Gray's Inn**
**London WC1R 5JJ (GB)**

(56) References cited:
**EP-A1- 1 184 379**
**EP-A1- 1 433 814**
**EP-A1- 1 927 477**
**WO-A1-2010/105289**
**WO-A1-2011/068110**
**JP-A- H04 216 807**
**JP-A- 2001 071 640**
**JP-A- 2009 079 090**
**JP-A- 2012 144 666**

- **HABICHER W D ET AL: "Synthesis and antioxidative properties of novel multifunctional stabilizers", JOURNAL OF VINYL AND ADDITIVE TECHNOLOGY, SOCIETY OF PLASTICS ENGINEERS, BROOKFIELD, CT, US, vol. 7, no. 1, 1 March 2001 (2001-03-01), pages 4-18, XP008104748, ISSN: 1083-5601, DOI: 10.1002/VNL.10258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 769 998 B1

**Description**

Technical Field:

This invention relates to a novel (meth) acrylate compound and to a novel curable composition that contains the (meth)acrylate compound and is useful for the production of optical articles having photochromic property.

Background Art:

Photochromic spectacles are the spectacles which, when used outdoors and irradiated with light that includes ultraviolet rays such as sunlight, work as sunglasses with their lenses being quickly colored and, when used indoors and are no longer irradiated with such light, work as ordinary spectacles with their color being faded. In recent years, demands for the photochromic spectacles are increasing.

As for the photochromic spectacle lenses, plastic lenses have been specifically preferred from the standpoint light weight and safety. Photochromic properties are, usually, imparted to the plastic lenses by compounding an organic photochromic compound and a polymerizable monomer or the lens together.

As the compounding method, there has been known a method of dissolving a photochromic compound in a polymerizable monomer and polymerizing them together to directly obtain a photochromic lens (hereinafter called in-mass method) (see a patent document 1).

There has, further, been known a method of forming a coating having photochromic property (hereinafter also called photochromic coating) on the surfaces of plastic lenses by using a coating agent containing a photochromic compound (hereinafter also called photochromic coating agent) (hereinafter called coating method) (see a patent document 2).

When the plastic lenses imparted with the photochromic property are used for extended periods of time, in general, the photochromic compound is gradually decomposed by light. Namely, problems occur from the standpoint of durability, such as a decrease in the color density, the lens develops yellow color, etc. To prevent such problems, means has been taken; i.e., a photo stabilizer as represented by a hindered amine compound is added.

However, the hindered amine compound which is the most generally used compound having a 2,2,6,6-tetramethyl-4-piperidyl group as represented by a sebacic acid (1,2,2,6,6-pentamethyl-4-piperidyl) diester, bleeds out gradually from the lens material or the coating though dependent on the amount it is added, and causes a problem in that the surfaces of the lens material or the coating become turbid. When the in-mass method is employed, in particular, the lens tends to be peeled off the glass mold (mold) in the step of curing by polymerization causing a problem in that the lens becomes defective since the surfaces of the lens are impaired by polymerization.

The above patent document 1, further, proposes the use of a (meth)acrylate compound having a 2,2,6,6-tetramethyl-4-piperidyl group and a (meth)acrylic group in a molecule, the 2, 2, 6, 6-tetramethyl-4-piperidyl group and the (meth) acrylic group being directly bonded together by ester. The (meth)acrylate compound chemically bonds in the cured body and does not cause the problem of bleed out. According to the study conducted by the present inventors, however, a problem was newly discovered in that the inherent effect for improving the durability is not exhibited to a sufficient degree and, besides, the photochromic property decreases and, specifically, the fading rate becomes slow.

Prior Art Documents:

Patent Documents:

Patent document 1: Pamphlet of WO2001/5854
Patent document 2: Pamphlet of WO2003/11967

Outline of the Invention:

Problems that the Invention is to Solve:

It is, therefore, an object of the present invention to provide a photochromic curable composition capable of producing photochromic lenses suppressing the problems of turbidity in the surfaces of the lenses caused by the hindered amine compound that bleeds out and peeling in the step of production, and imparting excellent durability and high photochromic property.

Means for Solving the Problems:

The present inventors have conducted keen study concerning the above problems. Concretely, the inventors have studied combining a compound having a 2,2,6,6-tetramethyl-4-piperidyl group with a photochromic compound. As a result, the inventors have discovered that a cured body free from the problem of the bleed out and excelling in photochromic properties could be obtained from a combination of a photochromic compound with a (meth)acrylate compound in which the 2,2,6,6-tetramethyl-4-piperidyl group and the (meth) acrylic group are bonded together via a specific divalent group, and have completed the present invention.

That is, a first invention is a photochromic curable composition that contains (A) a (meth)acrylate compound represented by the following formula (1), (B) a radically polymerizable monomer other than the (meth)acrylate compound represented by the formula (1), and (C) a photochromic compound,

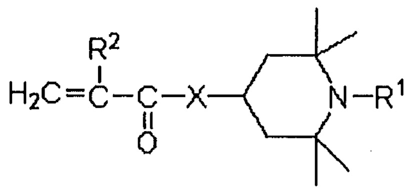

(1)

wherein,

$R^1$ and $R^2$ are, respectively, hydrogen atoms or methyl groups, and
X is a divalent group represented by the following formula (X1), (X2) or (X3),

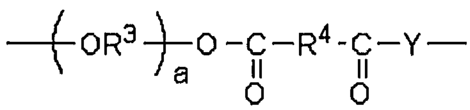

(X 1)

wherein,

$R^3$ is an alkylene group having 1 to 6 carbon atoms,
$R^4$ is an alkylene group having 1 to 6 carbon atoms, a cycloalkylene group having 3 to 8 carbon atoms and which may include a double bond, or an arylene group having 6 to 10 carbon atoms,
Y is an oxygen atom or a divalent group represented by -NH-, and
a is a positive number of 1 to 20,

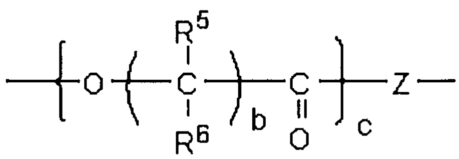

(X 2)

wherein,

$R^5$ and $R^6$ are, respectively, hydrogen atoms or methyl groups,
Z is an oxygen atom or a divalent group represented by -NH-,
b is a positive number of 2 to 7, and
c is a positive number of 1 to 20, or

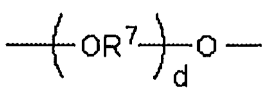

(X 3)

wherein,
$R^7$ is an alkylene group having 1 to 6 carbon atoms, and d is a positive number of 1 to 20.

To obtain the cured body having excellent photochromic properties and durability according to the first invention, it is desired that the photochromic curable composition contains (A) the (meth)acrylate compound represented by the above formula (1) in an amount of 0.01 to 20 parts by mass, (B) the radically polymerizable monomer other than the (meth)acrylate compound represented by the above formula (1) in an amount of 100 parts by mass, and (C) the photochromic compound in an amount of 0.01 to 20 parts by mass.

Further, it is desired that the above photochromic compound (C) contains a compound having an indeno[2,1-f]naphtho[1,2-b]pyran skeleton.

A second invention is a coating agent containing the above photochromic curable composition.

A third invention is an optical material having a photochromic coating obtained by curing the above coating agent on at least one surface of an optical base material.

A fourth invention is a photochromic cured body obtained by curing the above photochromic curable composition.

A fifth invention is a (meth)acrylate compound represented by the formula (1-X1);

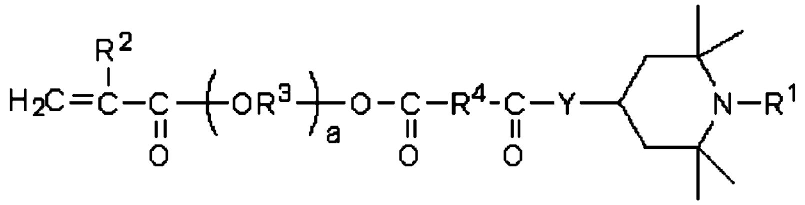

(1 - X 1)

wherein,

- $R^1$ and $R^2$ are, respectively, hydrogen atoms or methyl groups,
- $R^3$ is an alkylene group having 1 to 6 carbon atoms,
- $R^4$ is an alkylene group having 1 to 6 carbon atoms, a cycloalkylene group having 3 to 8 carbon atoms and which may include a double bond, or an arylene group having 6 to 10 carbon atoms,
- Y is an oxygen atom or a divalent group represented by -NH-, and
- a is a positive number of 1 to 20.

Effects of the Invention:

The cured body obtained by polymerizing and curing the photochromic curable composition of the present invention has the 2,2,6,6-tetramethyl-4-piperidyl group that is fixed by chemical bonding and is, therefore, free from the bleeding out. Accordingly, the lens material or the coating does not become turbid on the surfaces thereof, and does not permit the lens to peel off the glass mold during the curing by polymerization. As compared to the (meth)acrylate compound that has the 2,2,6,6-tetramethyl-4-piperidyl group and the (meth)acrylic group in a molecule thereof, the groups being directly bonded together via an ester, the photochromic cured body of the present invention exhibits highly improved durability and excellent photochromic property presumably due to a long molecular chain.

Modes for Carrying Out the Invention:

The present invention is the photochromic curable composition that contains (A) the (meth)acrylate compound represented by the above formula (1), (B) the radically polymerizable monomer other than the (meth)acrylate compound represented by the above formula (1), and (C) the photochromic compound. The components will now be described.

<Component A>

The present invention uses the (meth)acrylate compound in which the 2,2,6,6-tetramethyl-4-piperidyl group and the (meth)acrylic group are bonded together in a molecule via a specific divalent group. The (meth)acrylate compound is represented by the following formula (1) {the (meth)acrylate compound represented by the following formula (1) is hereinafter often referred to as the component (A)}.

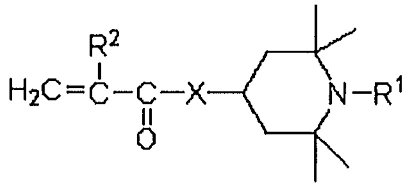

(1)

wherein,

$R^1$ and $R^2$ are, respectively, hydrogen atoms or methyl groups, and
X is a divalent group represented by the following formula (X1), (X2) or (X3),

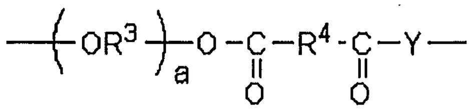

(X 1)

wherein,

$R^3$ is an alkylene group having 1 to 6 carbon atoms,
$R^4$ is an alkylene group having 1 to 6 carbon atoms, a cycloalkylene group having 3 to 8 carbon atoms and which may include a double bond, or an arylene group having 6 to 10 carbon atoms,
Y is an oxygen atom or a divalent group represented by -NH-, and
a is a positive number of 1 to 20,

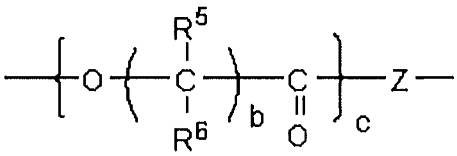

(X 2)

wherein,

$R^5$ and $R^6$ are, respectively, hydrogen atoms or methyl groups,
Z is an oxygen atom or a divalent group represented by -NH-,
b is a positive number of 2 to 7, and
c is a positive number of 1 to 20,
or

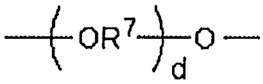

(X 3)

wherein,
$R^7$ is an alkylene group having 1 to 6 carbon atoms, and d is a positive number of 1 to 20.

The component (A) may be of a single kind or may consists of a plurality of kinds thereof.

It is considered that the cured body having excellent phorochromic property and durability is obtained owing to the above component (A) that has the divalent group represented by the above group (X1), (X2) or (X3).

[(Meth)acrylate compound having a group represented by the formula (X1)]

First, the component (A) having a divalent group represented by the formula (X1) will be described. In this case, the component (A) is a (meth) acrylate compound represented by the following formula (1-X1).

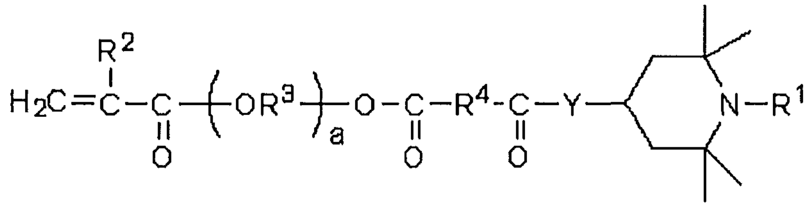

(1 - X 1)

wherein,

$R^1$ and $R^2$ are, respectively, hydrogen atoms or methyl groups,
$R^3$ is an alkylene group having 1 to 6 carbon atoms,
$R^4$ is an alkylene group having 1 to 6 carbon atoms, a cycloalkylene group having 3 to 8 carbon atoms and which may include a double bond, or an arylene group having 6 to 10 carbon atoms, Y is an oxygen atom or a divalent group represented by -NH-, and
a is a positive number of 1 to 20.

As the group represented by $R^1$, a methyl group is specifically preferred from the standpoint of excellent compatibility to the component (B) that will be described later and a high degree of durability.
As the alkylene group having 1 to 6 carbon atoms represented by $R^3$, there can be exemplified methylene group, ethylene group, n-propylene group, i-propylene group, n-butylene group, i-butylene group, n-pentylene group and n-hexylene group.
As the alkylene group having 1 to 6 carbon atoms represented by $R^4$, there can be exemplified the same groups as those exemplified for $R^3$.
As the cycloalkylene group having 3 to 8 carbon atoms and which may include one double bond, there can be exemplified cyclopropylene group, cyclobutylene group, cyclopentylene group, cyclohexylene group, cyclopeptylene group, cyclooctylene group and cyclohexenylene group.
As the arylene group having 6 to 10 carbon atoms, there can be exemplified phenylene group and naphthylene group.
Among them, preferred examples of $R^3$ are ethylene group, n-propylene group, i-propylene group and n-butylene group and preferred examples of $R^4$ are ethylene group, cyclohexylene group and cyclohexenylene group from the standpoint of obtaining particularly excellent durability.
Symbol a represents the number of $-OR^3-$ and is a positive number of 1 to 20. An increase in the number of a causes a decrease in the number of mols of the 2,2,6,6-tetramethylpiperidine skeletons contained in a unit mass of the (meth) acrylate compound. To improve the durability to a sufficient degree, therefore, the (meth) acrylate compound is often added in an increased amount causing, however, the moldability to be deteriorated. Therefore, a is a positive number of, desirably, 1 to 10 and, specifically, a positive number of 1 to 5.
When the (meth) acrylate compound represented by the above formula (1-X1) is used in the form of a mixture of a plurality of kinds thereof having different numbers of a, the average value of a of the mixture may be a positive number of 1 to 20 and, preferably, a positive number of 1 to 10 and, more preferably, a positive number of 1 to 5. When the mixture is used, however, it is desired that a of each of the (meth) acrylate compounds satisfies the range of 1 to 20.
As the (meth)acrylate compound represented by the above formula (1-X1), there can be exemplified the following compounds.

2-Acryloyloxyethylsuccinic acid
(2,2,6,6-tetramethyl-4-piperidyl) ester,
2-Acryloyloxyethylsuccinic acid
(1,2,2,6,6-pentamethyl-4-piperidyl) ester,
2-Methacryloyloxyethylsuccinic acid
(2,2,6,6-tetramethyl-4-piperidyl) ester,
2-Methacryloyloxyethylsuccinic acid
(1,2,2,6,6-pentamethyl-4-piperidyl) ester,
2-Acryloyloxypropylsuccinic acid
(1,2,2,6,6-pentamethyl-4-piperidyl) ester,
2-Acryloylpolyethoxy(a ≒ 2) succinic acid
(1,2,2,6,6-pentamethyl-4-piperidyl) ester,
2-Acryloylpolyethoxy(a ≒ 4.5) succinic acid
(1,2,2,6,6-pentamethyl-4-piperidyl) ester,

2-Acryloylpolyethoxy(a ≒ 8) succinic acid (1,2,2,6,6-pentamethyl-4-piperidyl) ester,
2-Methacryloylpolyethoxy(a ≒ 2) succinic acid (1,2,2,6,6-pentamethyl-4-piperidyl) ester,
2-Methacryloylpolyethoxy(a ≒ 4.5) succinic acid (1,2,2,6,6-pentamethyl-4-piperidyl) ester,
2-Methacryloylpolyethoxy(a ≒ 8) succinic acid (1,2,2,6,6-pentamethyl-4-piperidyl) ester,
2-Acryloyloxyethylsuccinic acid (1,2,2,6,6-pentamethyl-4-piperidyl) amide,
2-Acryloyloxyethylhexahydrophthalic acid (1,2,2,6,6-pentamethyl-4-piperidyl) ester,
2-Acryloyloxyethyl-1',2',5',6'-tetrahydrophthalic acid (1,2,2,6,6-pentamethyl-4-piperidyl) ester, and 2-Acryloyloxyethylphthalic acid (1,2,2,6,6-pentamethyl-4-piperidyl) ester.

[Method of producing the (meth)acrylate compound represented by the formula (1-X1)]

Though there is no specific limitation on the method of producing the (meth) acrylate compound represented by the above formula (1-X1), there can be exemplified the following method.

A compound represented by the following formula (2),

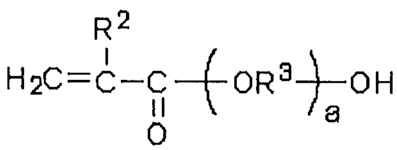
(2)

wherein,
$R^2$, $R^3$ and a are as defined in the above formula (1-X1), is subjected to the dehydration-condensation reaction with a compound represented by the following formula (3),

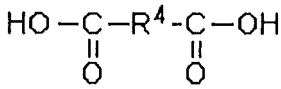
(3)

wherein,
$R^4$ is as defined in the above formula (1-X1), to obtain a compound represented by the following formula (4),

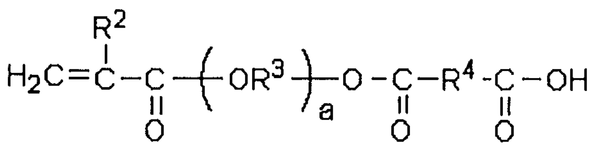
(4)

wherein,
$R^2$, $R^3$, $R^4$ and a are as defined in the above formula (1-X1).

Thereafter, the compound represented by the above formula (4) is subjected to the dehydration-condensation reaction with a compound represented by the following formula (5-1) or (5-2) to obtain the a (meth)acrylate compound represented by the formula (1-X1).

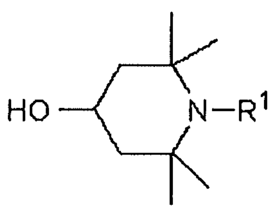
(5 − 1)

wherein,
$R^1$ is as defined in the above formula (1-X1).

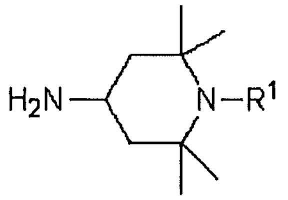

(5 - 2)

wherein,
$R^1$ is as defined in the above formula (1-X1).

As the dehydration-condensation reaction, there can be employed, for example, a method in which the above compounds are stirred at about room temperature to about 150°C for 1 to 48 hours with no solvent or being diluted with an inert solvent in the presence of an acid catalyst such as hydrochloric acid, sulfuric acid or p-toluenesulfonic acid. There can be, further, employed a method in which the above compounds are stirred with no solvent or being diluted with an inert solvent and being cooled with ice or at room temperature for 1 to 48 hours in the presence of a dehydration-condensation agent such as dicyclohexylcarbodiimide, diisopropylcarbodiimide or 1-ethyl-3-(3-dimethylaminopropyl)carbodiimide hydrochloride. As the inert solvent used for the above two reactions, there can be exemplified dichloromethane, chloroform, acetone, methyl ethyl ketone, tetrahydrofurane, toluene and xylene. In the case of the latter method, there can be used an additive such as 1-hydroxybenzotriazole, N-hydroxysuccinimide or N,N-dimethylaminopiridine in order to accelerate the reaction while suppressing the side reactions.

Further, the (meth) acrylate compound represented by the formula (1-X1) can be obtained by the following method, too. Namely, the compound represented by the above formula (2) is subjected to the condensation reaction with a compound represented by the following formula (3'),

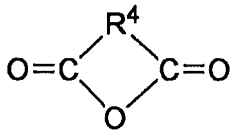

(3 ')

wherein,
$R^4$ is as defined in the above formula (1-X1), to obtain the compound represented by the above formula (4) which is then subjected to the dehydration-condensation reaction with the compound represented by the above formula (5-1) or (5-2).

As the condensation reaction in this case, the compounds are stirred at about room temperature to about 150°C for 1 to 48 hours without solvent or being diluted with an inert solvent such as N, N-dimethylformamide in the presence of a base catalyst such as pyridine.

As another production method, the compound represented by the above formula (3') is subjected to the condensation reaction with the compound represented by the above formula (5-1) or (5-2) to obtain a compound represented by the following formula (6-1) or (6-2),

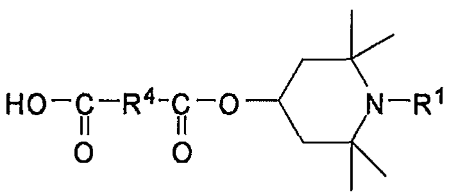

(6 - 1)

wherein,
$R^1$ and $R^4$ are as defined in the above formula (1-X1), or

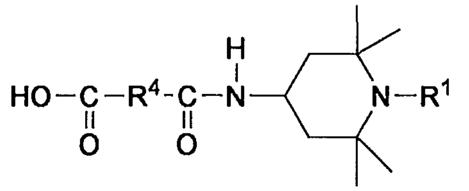

(6 − 2)

wherein,
$R^1$ and $R^4$ are as defined in the above formula (1-X1), which compound is then subjected to the dehydration-condensation reaction with the compound represented by the above formula (2) to obtain the compound represented by the above formula (1-X1).

Concrete methods of the condensation reaction and the dehydration-condensation reaction are as described above.

As the compound represented by the above formula (2), there can be used, for example, the following compounds that have been placed in the market.

Hydroxyethyl acrylate,
Hydroxyethyl methacrylate,
Polyethylene glycol (a ≒ 2) monoacrylate
(Blenmar AE-90, manufactured by Nichiyu Co.),
Polyethylene glycol (a ≒ 4.5) monoacrylate
(Blenmar AE-200, manufactured by Nichiyu Co.),
Polyethylene glycol (a ≒ 8) monoacrylate
(Blenmar AE-350, manufactured by Nichiyu Co.),
Polyethylene glycol (a ≒ 2) monomethacrylate (Blenmar PE-90, manufactured by Nichiyu Co.),
Polyethylene glycol (a ≒ 4.5) monomethacrylate
(Blenmar PE-200, manufactured by Nichiyu Co.),
Polyethylene glycol (a ≒ 8) monomethacrylate
(Blenmar PE-350, manufactured by Nichiyu Co.),
Hydroxypropylmethacrylate
(Blenmar P, manufactured by Nichiyu Co.),
Polypropylene glycol (a ≒ 6) monoacrylate
(Blenmar AP-400, manufactured by Nichiyu Co.),
Polypropylene glycol (a ≒ 9) monoacrylate
(Blenmar AP-550, manufactured by Nichiyu Co.),
Polypropylene glycol (a ≒ 13) monoacrylate
(Blenmar AP-800, manufactured by Nichiyu Co.),
Polypropylene glycol (a ≒ 9) monomethacrylate
(Blenmar PP-500, manufactured by Nichiyu Co.),
Polypropylene glycol (a ≒ 13) monomethacrylate
(Blenmar PP-800, manufactured by Nichiyu Co.),
Polypropylene glycol (a ≒ 16) monomethacrylate (Blenmar PP-1000, manufactured by Nichiyu Co.).

As the compound represented by the above formula (4), there can be used, for example, the following compounds that have been placed in the market.

2-Methacryloyloxyethylsuccinic acid (NK Ester SA,
manufactured by Shin-Nakamura Kagaku Kogyo Co.),
2-Acryloyloxyethylsuccinic acid (NK Ester A-SA,
manufactured by Shin-Nakamura Kagaku Kogyo Co.),
2-Acryloyloxyethylhexahydrophthalic acid (HOA-HH,
manufactured by Kyoeisha Co.),
2-Acryloyloxyethylphthalic acid (Alonics M-5400, manufactured by Toa Gosei Kagaku Co.).

[(Meth)acrylate compound having a group represented by the above formula (X2)]

Of the components (A), the (meth) acrylate compound having a divalent group represented by the above formula

(X2) is represented by the following formula (1-X2),

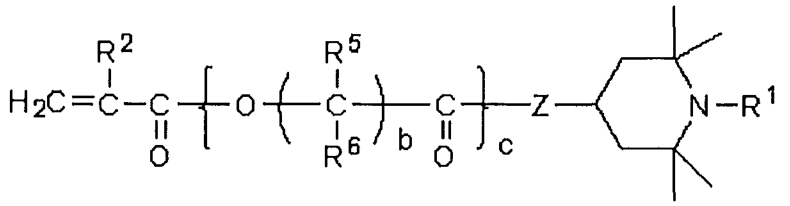

(1 - X 2)

wherein,

$R^1$, $R^2$, $R^5$ and $R^6$ are, respectively, hydrogen atoms or methyl groups,
Z is an oxygen atom or a divalent group represented by -NH-,
b is a positive number of 2 to 7, and
c is a positive number of 1 to 20.

Symbol b is the positive number of 2 to 7. From the standpoint of easily obtaining the starting material, however, it is desired that b is 4 to 6. When the (meth) acrylate compound represented by the above formula (1-X2) is used in the form of a mixture of a plurality of kinds thereof having different numbers of b, the average value of b of the mixture may be a positive number of 2 to 7 and, preferably, a positive number of 4 to 6. When the mixture is used, however, it is desired that b of each of the (meth)acrylate compounds satisfies the range of 2 to 7.

Symbol c is the positive number of 1 to 20. An increase in the number of c causes a decrease in the number of mols of the 2,2,6,6-tetramethylpiperidine skeletons contained in a unit mass of the (meth)acrylate compound. To improve the durability to a sufficient degree, therefore, the (meth)acrylate compound is often added in an increased amount causing, however, the moldability to be deteriorated. Therefore, c is a positive number of, desirably, 1 to 10 and, specifically, a positive number of 1 to 5.

Here, when the (meth) acrylate compound represented by the above formula (1-X2) is used in the form of a mixture of a plurality of kinds thereof having different numbers of c, the average value of c of the mixture may be a positive number of 1 to 20 and, preferably, a positive number of 1 to 10 and, more preferably, a positive number of 1 to 5. When the mixture is used, however, it is desired that c of each of the (meth) acrylate compounds satisfies the range of 1 to 20.

As the group represented by $R^1$, a methyl group is specifically preferred from the standpoint of excellent compatibility to the component (B) that will be described later and a high degree of durability.

As the(meth)acrylate compound represented by the above formula (1-X2), there can be exemplified the following compounds.

Acryloyloxypolycaprolactone (c ≒ 2) carboxylic acid (2,2,6,6-tetramethyl-4-piperidyl) ester,
Acryloyloxypolycaprolactone (c ≒ 2) carboxylic acid (1,2,2,6,6-pentamethyl-4-piperidyl) ester,
Acryloyloxypolycaprolactone (c ≒ 4) carboxylic acid (2,2,6,6-tetramethyl-4-piperidyl) ester,
Acryloyloxypolycaprolactone (c ≒ 4) carboxylic acid (1,2,2,6,6-pentamethyl-4-piperidyl) ester,
Acryloyloxypolycaprolactone (c ≒ 8) carboxylic acid (2,2,6,6-tetramethyl-4-piperidyl) ester,
Acryloyloxypolycaprolactone (c ≒ 8) carboxylic acid (1,2,2,6,6-pentamethyl-4-piperidyl) ester,
Acryloyloxypolycaprolactone (c ≒ 20) carboxylic acid (2,2,6,6-tetramethyl-4-piperidyl) ester,
Acryloyloxypolycaprolactone (c ≒ 20) carboxylic acid (1,2,2,6,6-pentamethyl-4-piperidyl) ester,
Methacryloyloxypolycaprolactone (c ≒ 2) carboxylic acid (2,2,6,6-tetramethyl-4-piperidyl) ester,
Methacryloyloxypolycaprolactone (c ≒ 2) carboxylic acid (1,2,2,6,6-pentamethyl-4-piperidyl) ester,
Methacryloyloxypolycaprolactone (c ≒ 4) carboxylic acid (2,2,6,6-tetramethyl-4-piperidyl) ester,
Methacryloyloxypolycaprolactone (c ≒ 4) carboxylic acid (1,2,2,6,6-pentamethyl-4-piperidyl) ester,
Methacryloyloxypolycaprolactone (c ≒ 8) carboxylic acid (2,2,6,6-tetramethyl-4-piperidyl) ester,
Methacryloyloxypolycaprolactone (c ≒ 8) carboxylic acid (1,2,2,6,6-pentamethyl-4-piperidyl) ester,
Methacryloyloxypolycaprolactone (c ≒ 20) carboxylic acid (2,2,6,6-tetramethyl-4-piperidyl) ester,
Methacryloyloxypolycaprolactone (c ≒ 20) carboxylic acid (1,2,2,6,6-pentamethyl-4-piperidyl) ester,
Acryloyloxypolycaprolactone (c ≒ 2) carboxylic acid (1,2,2,6,6-pentamethyl-4-piperidyl) amide,
Methacryloyloxypolycaprolactone (c ≒ 2) carboxylic acid (1,2,2,6,6-pentamethyl-4-piperidyl) amide,
Acryloyloxypolybutylolactone (c ≒ 2) carboxylic acid (1,2,2,6,6-pentamethyl-4-piperidyl) ester,
Methacryloyloxypolybutylolactone (c ≒ 2) carboxylic acid (1,2,2,6,6-pentamethyl-4-piperidyl) ester,
Acryloyloxypolybutylolactone (c ≒ 2) carboxylic acid (1,2,2,6,6-pentamethyl-4-piperidyl) amide,

Methacryloyloxypolybutylolactone (c ≒ 2) carboxylic acid (1,2,2,6,6-pentamethyl-4-piperidyl) amide,
Acryloyloxypolyvalerolactone (c ≒ 2) carboxylic acid (1,2,2,6,6-pentamethyl-4-piperidyl) ester,
Methacryloyloxypolyvalerolactone (c ≒ 2) carboxylic acid (1,2,2,6,6-pentamethyl-4-piperidyl) ester,
Acryloyloxypolyvalerolactone (c ≒ 2) carboxylic acid (1,2,2,6,6-pentamethyl-4-piperidyl) amide, and
Methacryloyloxypolyvalerolactone (c ≒ 2) carboxylic acid (1,2,2,6,6-pentamethyl-4-piperidyl) amide.

[Method of producing the (meth)acrylate compound represented by the formula (1-X2)]

Though there is no specific limitation on the method of producing the (meth) acrylate compound represented by the above formula (1-X2), there can be exemplified the following method.

A compound represented by the following formula (7),

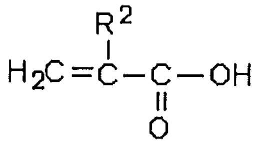

(7)

wherein,
$R^2$ is as defined in the above formula (1-X2),
is subjected to the ring-opening addition polymerization with lactones represented by the following formula (8),

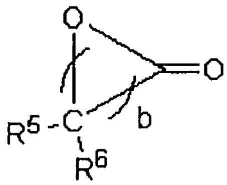

(8)

wherein,
$R^5$, $R^6$ and b are as defined in the above formula (1-X2), at 90 to 240°C in the presence of a catalyst to obtain a compound represented by the following formula (9),

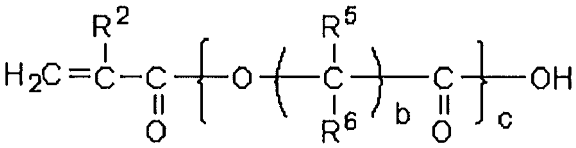

(9)

wherein,
$R^2$, $R^5$, $R^6$, b and c are as defined in the above formula (1-X2).

Thereafter, the compound represented by the formula (9) and the compound represented by the above formula (5-1) or (5-2) are subjected to the dehydration-condensation reaction to produce the compound represented by the formula (1-X2). As the dehydration-condensation reaction, there can be exemplified the same method as the one described in the production of the compound of the above formula (1-X1).

In the reaction for obtaining the compound represented by the formula (9) from the compound represented by the formula (7) and the compound represented by the formula (8), there is used the catalyst, i.e.,:

organotitanium type compound such as tetraethyl titanate, tetrabutyl titanate or tetrapropyl titanate;
organotin compound such as stannous octylate, dibutyltin oxide, dibutyltin dilaurate or mono-n-butyltin fatty acid salt; or
stannous halide such as stannous chloride, stannous bromide or stannous iodide.

As the compound represented by the above formula (8), there can be concretely exemplified,

ε-Caprolactone,
Trimethyl-E-caprolactone,
Monomethyl-ε-caprolactone,

γ-Butylolactone, and
δ-Valerolactone.

As the compound represented by the above formula (9), there can be used, for example, an acryloyloxypolycaprolactone (c ≒ 2) carboxylic acid (Alonics M-5300, manufactured by Toa Gosei Kagaku Co.) that has been placed in the market.

[(Meth)acrylate compound having a group represented by the formula (X3)]

Of the components (A), the (meth) acrylate compound having a divalent group represented by the above formula (X3) is expressed by the following formula (1-X3),

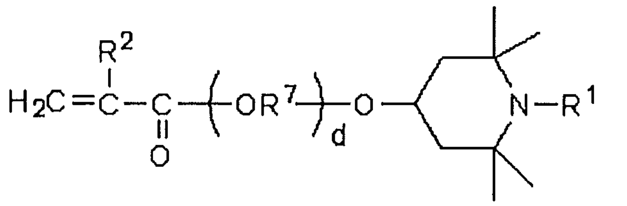

(1 - X 3)

wherein,

$R^1$ and $R^2$ are, respectively, hydrogen atoms or methyl groups,
$R^7$ is an alkylene group having 1 to 6 carbon atoms, and d is a positive number of 1 to 20.

As the group represented by $R^1$, a methyl group is specifically desired from the standpoint of excellent compatibility to the component (B) that will be described later and a high degree of durability.

As the alkylene group having 1 to 6 carbon atoms represented by $R^7$, there can be exemplified methylene group, ethylene group, n-propylene group, i-propylene group, n-butylene group, n-pentylene group and n-hexylene group. Among them, specifically preferred are the ethylene group, n-propylene group, i-propylene group and n-butylene group from the standpoint of excellent durability.

Symbol d is a positive number of 1 to 20. An increase in the number of d causes a decrease in the number of mols of the 2,2,6,6-tetramethylpiperidine skeletons contained in a unit mass of the (meth)acrylate compound. To improve the durability to a sufficient degree, therefore, the (meth)acrylate compound is often added in an increased amount causing, however, the moldability to be deteriorated. Therefore, d is a positive number of, desirably, 1 to 10 and, specifically, a positive number of 2 to 5.

When the (meth) acrylate compound represented by the above formula (1-X3) is used in the form of a mixture of a plurality of kinds thereof having different numbers of d, the average value of d of the mixture may be a positive number of 1 to 20 and, preferably, a positive number of 1 to 10 and, more preferably, a positive number of 1 to 5. When the mixture is used, however, it is desired that d of each of the (meth) acrylate compounds satisfies the range of 1 to 20.

As the (meth)acrylate compound represented by the above formula (1-X3), there can be exemplified the following compounds. 2,2,6,6-Tetramethyl-4-piperidyl-ethoxy acrylate, 1,2,2,6,6-Pentamethyl-4-piperidyl-ethoxy acrylate, 2,2,6,6-Tetramethyl-4-piperidyl-ethoxy methacrylate, 1,2,2,6,6-Pentamethyl-4-piperidyl-ethoxy methacrylate, 2,2,6,6-Tetramethyl-4-piperidyl-diethoxy acrylate, 1,2,2,6,6-Pentamethyl-4-piperidyl-diethoxy acrylate, 2,2,6,6-Tetramethyl-4-piperidyl-diethoxy methacrylate, 1,2,2,6,6-Pentamethyl-4-piperidyl-diethoxy methacrylate, 2,2,6,6-Tetramethyl-4-piperidyl-polyethoxy (d ≒ 6) acrylate, 1,2,2,6,6-Pentamethyl-4-piperidyl-polyethoxy (d ≒ 6) acrylate, 2,2,6,6-Tetramethyl-4-piperidyl-polyethoxy (d ≒ 6) methacrylate, 1,2,2,6,6-Pentamethyl-4-piperidyl-polyethoxy (d ≒ 6) methacrylate, 1,2,2,6,6-Pentamethyl-4-piperidyl-polyethoxy (d ≒ 10) acrylate, 1,2,2,6,6-Pentamethyl-4-piperidyl-polyethoxy (d ≒ 10) methacrylate, 1,2,2,6,6-Pentamethyl-4-piperidyl-polyethoxy (d ≒ 20) acrylate, 1,2,2,6,6-Pentamethyl-4-piperidyl-polyethoxy (d ≒ 20) methacrylate, 1,2,2,6,6-Pentamethyl-4-piperidyl-i-propoxy acrylate, 1,2,2,6,6-Pentamethyl-4-piperidyl-i-propoxy methacrylate, 1,2,2,6,6-Pentamethyl-4-piperidyl-poly i-propoxy (d ≒ 6) acrylate, 1,2,2,6,6-Pentamethyl-4-piperidyl-poly i-propoxy (d ≒ 6) methacrylate, 1,2,2,6,6-Pentamethyl-4-piperidyl-n-butoxy acrylate, and 1,2,2,6,6-Pentamethyl-4-piperidyl-n-butoxy methacrylate.

The method of synthesizing the compounds of the above formula (1-X3) has been disclosed in, for example, JP-A-2001-71640.

[Preferred components (A)]

Of the compounds represented by the above formulas (1-X1), (1-X2) and (1-X3), preferred is a compound containing a carbonyl group which is a divalent group bonding the 2,2,6,6-tetramethyl-4-piperidyl group to the (meth)acrylate group, i.e., the compound represented by the above formula (1-X1) or (1-X2) from the standpoint of obtaining particularly excellent durability. From the standpoint of excellent compatibility to the component (B) that will be described later and easy availability of the starting material, further, the compound represented by the above formula (1-X1) is specifically desired.

<Component (B)>

The invention uses a radically polymerizable monomer {hereinafter often referred to as the component (B) } other than the above component (A). Though there is no specific limitation, the component (B) used for the photochromic curable composition of the invention will be, for example, a monofunctional (meth)acrylate compound, a bifunctional (meth)acrylate compound or a trifunctional or more highly functional (meth)acrylate compound, vinyl compound and allyl compound. These compounds may be used in a plurality of kinds depending on the use of the cured body.
Concretely, there can be preferably used radically polymerizable monomers disclosed in a pamphlet of WO2001/005854, pamphlet of WO2003/11967, pamphlet of WO2004/83268 and a pamphlet of WO2009/75388,
**[0066]** As the monofunctional (meth) acrylate compound, there can be used a compound represented by the following formula (10),

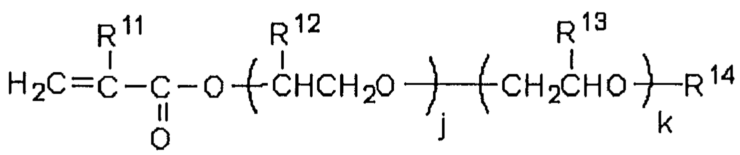 (10)

wherein,

$R^{11}$, $R^{12}$ and $R^{13}$ are, respectively, hydrogen atoms or methyl groups,
$R^{14}$ is a hydrogen atom, an alkyl group having 1 to 20 carbon atoms, a cycloalkyl group having 6 to 20 carbon atoms, a phenyl group which is unsubstituted or is substituted with an alkyl group having 1 to 20 carbon atoms, a naphthyl group which is unsubstituted or is substituted with an alkyl group having 1 to 20 carbon atoms, or a glycidyl group,
j is 0 to 25 and k is 0 to 25 under a condition that an average value of j + k is not less than 0 but is not more than 25.

The compound represented by the above formula (10) is, usually, obtained in the form of a mixture of molecules of different molecular weights. Therefore, the values of j and k are their average values, j being 0 to 25 and k being 0 to 25. In the monofunctional polymerizable monomer, it is desired that the average value of j + k is not less than 0 but is not more than 25 and, specifically, is not less than 0 but is not more than 15.
Described below are examples of the monofunctional polymerizable monomer that can be particularly preferably used. Methoxydiethylene glycol methacrylate, Methoxytetraethylene glycol methacrylate, Isostearyl methacrylate, Isobornyl methacrylate, Phenoxyethylene glycol methacrylate, Phenoxyethyl acrylate, Phenoxydiethylene glycol acrylate, Naphthoxyethylene glycol acrylate, Isotearyl acrylate, Isobornyl acrylate, Glycidyl methacrylate, Methoxypolyethylene glycol methacrylate (average length of ethylene glycol chain, 9; average molecular weight, 468), Methoxypolyethylene glycol methacrylate (average length of ethylene glycol chain, 23; average molecular weight, 1068), and Phenoxypolyethylene glycol acrylate (average length of ethylene glycol chain, 6; average molecular weight, 412).
These monofunctional radically polymerizable monomers may be used being mixed together in two or more kinds.
As the monofunctional (meth)acrylate compound, there can be, further, used a compound of the following formula (11),

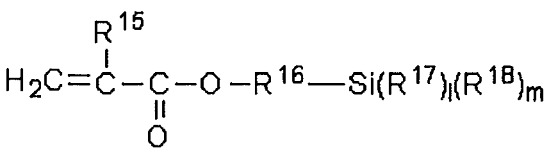 (11)

wherein, $R^{15}$ is a hydrogen atom or a methyl group, $R^{16}$ is an alkylene group having 1 to 10 carbon atoms, $R^{17}$ is an

alkoxy group having 1 to 6 carbon atoms, $R^{18}$ is an alkyl group having 1 to 6 carbon atoms, and 1 is an integer of 1 to 3 and m is an integer of 0 to 2 under a condition that l + m = 3.

As the alkylene group having 1 to 10 carbon atoms, there can be exemplified ethylene group, propylene group and butylene group.

As the alkoxy group having 1 to 6 carbon atoms, there can be exemplified methoxy group, ethoxy group and propoxy group.

As the alkyl group having 1 to 6 carbon atoms, there can be exemplified methyl group, ethyl group and propyl group.

As the monofunctional radically polymerizable monomer represented by the above formula (11) and that can be preferably used, there can be exemplified the following monomers. γ-Methacryloyloxypropyltrimethoxysilane, γ-Methacryloyloxypropyltriethoxysilane, and γ-Methacryloyloxypropylmethyldimethoxysilane.

These monofunctional radically polymerizable monomers may be used being mixed together in two or more kinds.

As the bifunctional (meth)acrylate compound, there can be used a compound represented by the following formula (12),

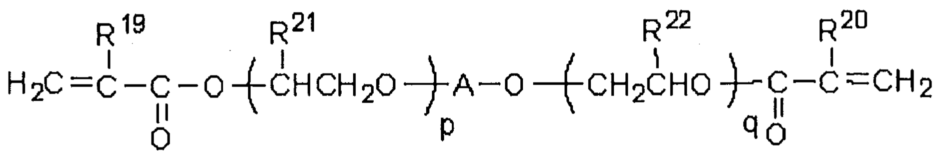 (12)

wherein,

p is 0 to 30 and q is 0 to 30 under a condition that an average value of p + q is not less than 0 but is not more than 30,
$R^{19}$, $R^{20}$, $R^{21}$ and $R^{22}$ are, respectively, hydrogen atoms or methyl groups,
A is a divalent organic group selected from the following group under a condition that the number of carbon atoms is 1 to 20,
alkylene group,
unsubstituted phenylene group,
phenylene group substituted with a halogen atom or
an alkyl group having 1 to 4 carbon atoms, and
a group represented by the following formula (12a), (12b) or (12c),

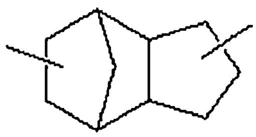 (12a)

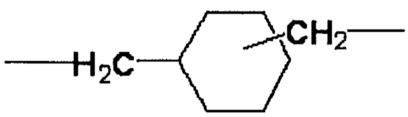 (12b)

or

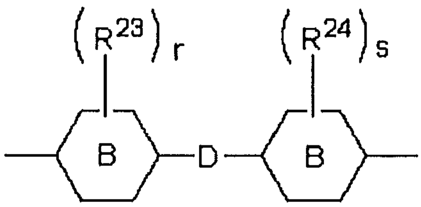 (12c)

wherein,

$R^{23}$ and $R^{24}$ are, respectively, alkyl groups having 1 to 4 carbon atoms or halogen atoms,

r and s are, respectively, integers of 0 to 4, six-membered rings B are benzene rings or cyclohexane rings and when the six-membered rings B are benzene rings, D is any group selected from the group consisting of -O-, -S-, $-S(O_2)-$, -C(O)-, $-CH_2-$, -CH=CH-, $-C(CH_3)_2-$ and $-C(CH_3)(C_6H_5)-$ or a group represented by the following formula (12c-1),

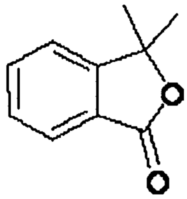

(12c－1)

and when the six-membered rings B are cyclohexane rings, D is any group selected from the group consisting of -O-, -S-, $-CH_2-$ and $-C(CH_3)_2-$.

The bifunctional radically polymerizable monomer represented by the above formula (12) is, usually, obtained in the form of a mixture of molecules of different molecular weights. Therefore, the values of p and q are their average values, p being 0 to 30, q being 0 to 30, and the average value of p + q being not less than 0 but is not more than 30.

As the alkylene group A in the above formula (12), there can be exemplified ethylene group, propylene group, butylene group and nonylene group.

As the phenylene group A substituted with a halogen atom or an alkyl group having 1 to 4 carbon atoms, there can be exemplified dimethylphenyl group, tetramethylphenyl group, dibromophenyl group and tetrabromophenyl group.

The following compounds can be exemplified as the bifunctional (meth) acrylate compound represented by the above formula (12) that can be preferably used.

Ethylene glycol dimethacrylate,
Diethylene glycol dimethacrylate,
Triethylene glycol dimethacrylate,
Tetraethylene glycol dimethacrylate,
Polyethylene glycol dimethacrylate (average length of ethylene glycol chain, 9; average molecular weight, 536),
Polyethylene glycol dimethacrylate (average length of ethylene glycol chain, 14; average molecular weight, 736),
Polyethylene glycol dimethacrylate (average length of ethylene glycol chain, 23; average molecular weight, 1136),
Tripropylene glycol dimethacrylate,
Tetrapropylene glycol dimethacrylate,
Polypropylene glycol dimethacrylate (average length of propylene glycol chain, 9; average molecular weight, 662),
Ethylene glycol diacrylate,
Diethylene glycol diacrylate,
Triethylene glycol diacrylate,
Tetraethylene glycol diacrylate,
Polyethylene glycol diacrylate (average length of ethylene glycol chain, 9; average molecular weight, 508),
Polyethylene glycol diacrylate (average length of ethylene glycol chain, 14; average molecular weight, 708),
Dipropylene glycol diacrylate,
Tripropylene glycol diacrylate,
Tetrapropylene glycol diacrylate,
Polypropylene glycol diacrylate (average length of propylene glycol chain, 7; average molecular weight, 536),
Polypropylene glycol diacrylate (average length of propylene glycol chain, 12; average molecular weight, 808),
1,3-Butanediol dimethacrylate,
1,6-Hexanediol dimethacrylate,
1,9-Nonanediol dimethacrylate,
1,10-Decanediol dimethacrylate,
Neopentyl glycol dimethacrylate,
2,2-Bis[4-methacryloxy(polyethoxy)phenyl]propane (average value of p + q, 2.3),
2,2-Bis[4-methacryloxy(polyethoxy)phenyl]propane (average value of p + q, 2.6),
2,2-Bis[4-methacryloxy(polyethoxy)phenyl]propane (average value of p + q, 4),
2,2-Bis[4-methacryloxy(polyethoxy)phenyl]propane (average value of p + q, 10),
2,2-Bis[4-methacryloxy(polyethoxy)phenyl]propane (average value of p + q, 20),
2,2-Bis[4-methacryloxy(polyethoxy)phenyl]propane (average value of p + q, 30),

Tricyclodecanedimethanol dimethacrylate,
1,6-Hexanediol diacrylate,
1,9-Nonanediol diacrylate,
1,10-Decanediol diacrylate
Neopentyl glycol diacrylate,
Tricyclodecanedimethanol diacrylate,
Dioxane glycol diacrylate,
Ethoxylated cyclohexanedimethanol diacrylate (average value of p + q, 4),
2,2-Bis[4-acryloxy(polyethoxy)phenyl]propane (average value of p + q, 3),
2,2-Bis[4-acryloxy(polyethoxy)phenyl]propane (average value of p + q, 4), and
2,2-Bis[4-acryloxy(polyethoxy)phenyl]propane (average value of p + q, 10).

These radically polymerizable monomers may be used being mixed in two or more kinds together.

As the bifunctional (meth)acrylate compound, further, there can be preferably used a bifunctional (meth)acrylate compound having a urethane bond in the molecules thereof, such as known urethane (meth)acrylates. The urethane (meth)acrylates can be roughly divided into those having an aromatic ring such as benzene ring in the molecular structure thereof and those having no aromatic ring. Either one of them can be used in the present invention. From the standpoint of light resistance of the cured body, however, it is desired to use the one of the type that has no aromatic ring and that does not develop yellow color.

As the urethane (meth) acrylate, there can be exemplified a reaction mixture obtained by reacting a diisocyanate with a polyol to form an urethane prepolymer which is, further, reacted with a 2-hydroxyethyl (meth) acrylate that may have an alkylene oxide chain, or a reaction mixture obtained by reacting the diisocyanate directly with the 2-hydroxyethyl (meth)acrylate that may have the alkylene oxide chain, having molecular weight not less than 400 but less than 20,000.

As the diisocyanate used for producing the urethane prepolymer, there can be exemplified,

Hexamethylene diisocyanate,
Isophorone diisocyanate,
Lizine isocyanate,
2,2,4-Trimethylhexamethylene diisocyanate,
5 Diisocyanate dimerate,
Isopropyridenebis-4-cyclohexyl isocyanate,
Dicyclohexylmethane diisocyanate,
Norbornene diisocyanate, and
Methylcyclohexane diisocyanate.

Further, the polyols to be reacted with the diisocyanate can be roughly divided into those of high molecular weights and low molecular weights. Of them, the polyols of high molecular weights may include:

Polyalkylene glycols having a recurring unit of 2 to 6 carbon atoms, such as ethylene oxide, propylene oxide and hexamethylene oxide;
Polyester diols such as polycaprolactone diol and the like;
Polycarbonate diols; and
Polybutadiene diols.

As the polyols of low molecular weights, there can be exemplified known polyols, such as ethylene glycol, propylene glycol, 1,3-propane diol, 1,4-butane diol, 1,5-pentane diol, 1.6-hexane diol, 1,9-nonane diol, 1,8-nonane diol, neopentyl glycol, diethylene glycol, dipropylene glycol, 1,4-cyclohexane diol, and 1,4-cyclohexane dimethanol.

As the trifunctional or more highly functional (meth)acrylate compound, there can be used a compound represented by the following formula (1-3),

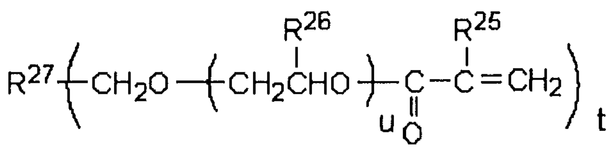
(13)

wherein,

$R^{25}$ and $R^{26}$ are, respectively, hydrogen atoms or methyl groups,
$R^{27}$ is an organic group having 1 to 10 carbon atoms and a valence of 3 to 6,
u is a number of 0 to 3 on the average, and t is an integer of 3 to 6.

Concretely, there can be exemplified the following compounds, Trimethylolpropane trimethacrylate, Trimethylolpropane triacrylate, Tetramethylolmethane trimethacrylate, Tetramethylolmethane triacrylate, Tetramethylolmethane tetramethacrylate, Tetramethylolmethane tetraacrylate, Trimethylolpropanetriethylene glyol trimethacrylate, Trimethylolpropanetriethylene glyol triacrylate, Ditrimethylolpropane tetramethacrylate, Ditrimethylolpropane tetraacrylate, Polyester oligomer having 4 (meth)acrylic groups, Polyester oligomer having 6 (meth)acrylic groups, Polyurethane oligomer having 4 (meth) acrylic groups, and Polyurethane oligomer having 6 (meth)acrylic groups.
As other trifunctional or more highly functional (meth)acrylate compounds, there can be exemplified silsesquioxanes having three or more (meth)acrylic groups. Concretely, there can be used a compound (silsesquioxane) represented by the following formula (14),

$$\left( R^{28}\text{-}SiO_{3/2} \right)_v \qquad (14)$$

wherein,

v represents a polymerization degree and is a positive number of 6 to 100, and
a plurality of $R^{28}$ may be the same or different, at least three $R^{28}$ being organic groups having a (meth)acrylic group, and
other $R^{28}$ than the organic groups having a (meth) acrylic group being hydrogen atoms, alkyl groups, cycloalkyl groups, alkoxy groups, halogenated alkyl groups, phenyl groups, phenyl groups substituted with halogen or hydroxyl groups.

Concretely, there can be exemplified the following compounds.
AC-SQ TA-100:

Polyacryloxypropylpolyorganosiloxane (a mixture of cage-like silsesquioxane having 8 acrylic groups in a molecule, a ladder-like silsesquioxane having 3 or more acrylic groups in a molecule, etc.) (produced by Toa Gosei Co.).

MAC-SQ TM-100:

Polymethacryloxypropylpolyorganosiloxane (a mixture of cage-like silsesquioxane having 8 methacrylic groups in a molecule, a ladder-like silsesquioxane having 3 or more methacrylic groups in a molecule, etc.) (produced by Toa Gosei Co.).

MA0735:

Polymethacryloxypropylpolyorganosiloxane compound (a mixture of cage-like silsesquioxanes having 8, 10 and 12 methacrylic groups in a molecule) (produced by Hybrid Plastics Co.).

As other silsesquioxanes having three or more (meth) acrylic groups in a molecule, further, there can be used those having the ladder structure and those having both the cage structure and the ladder structure.
Further, as the other radically polymerizable monomers, there can be used vinyl or allyl compounds such as styrene, $\alpha$ -methylstyrene, $\alpha$-methylstyrene dimer, divinylbenzene and allyl diglycol carbonate.
There is no specific limitation on the ratios of adding the monofunctional (meth)acrylate, bifunctional (meth)acrylate, trifunctional or more highly functional (meth) acrylate and vinyl or allyl compound in 100 parts by mass of the component (B). From the standpoint of attaining a high color density and a fast fading rate, however, it is desired that the ratios of addition are 0 to 20 parts by mass of the monofunctional (meth)acrylate, 30 to 90 parts by mass of the bifunctional (meth)acrylate, 0 to 60 parts by mass of the trifunctional or more highly functional (meth)acrylate, and 0 to 20 parts by mass of the vinyl or allyl compound.
More desirably, the ratios of addition are 0 to 20 parts by mass of the monofunctional (meth)acrylate, 30 to 90 parts by mass of the bifunctional (meth)acrylate, 0 to 40 parts by mass of the trifunctional or more highly functional (meth) acrylate, and 0 to 20 parts by mass of the vinyl or allyl compound.

Specifically desirably, the ratios of addition are 0 to 10 parts by mass of the monofunctional (meth)acrylate, 50 to 90 parts by mass of the bifunctional (meth)acrylate, 5 to 30 parts by mass of the trifunctional or more highly functional (meth) acrylate, and 0 to 10 parts by mass of the vinyl or allyl compound.

<Component (C)>

Next, described below is the photochromic compound {hereinafter often referred to as the component (C)} that is favorably used in the invention.

The photochromic curable composition of the present invention can use known photochromic compounds such as chromene compound, fulgimide compound, spirooxazine compound and spiropyran compound without any limitation. These compounds may be used in a single kind or in a combination of two or more kinds.

As the chromene compound, fulgimide compound, spirooxazine compound and spiropyran compound, there can be exemplified the compounds that have been disclosed in, for example,

JP-A-2-28154,
JP-A-62-288830,
Pamphlet of WO94/22850, and
Pamphlet of WO96/14596.

Specifically, in addition to the chromene compounds described in the above patent documents, there have, further, been known chromene compounds having excellent photochromic properties, which can also be used in the present invention. Such chromene compounds have been disclosed, for example, in the following documents.

JP-A-2001-031670,
JP-A-2001-011067,
JP-A-2001-011066,
JP-A-2000-344761,
JP-A-2000-327675,
JP-A-2000-256347,
JP-A-2000-229976,
JP-A-2000-229975,
JP-A-2000-229974,
JP-A-2000-229973,
JP-A-2000-229972,
JP-A-2000-219678,
JP-A-2000-219686,
JP-A-11-322739,
JP-A-11-286484,
JP-A-11-279171,
JP-A-09-218301,
JP-A-09-124645
JP-A-08-295690
JP-A-08-176139
JP-A-08-157467,
USP5645767,
USP5658501,
USP5961892,
USP6296785,
JP4424981,
JP4424962,
Pamphlet of WO2009/136668,
Pamphlet of WO2008/023828,
JP4369754,
JP4301621,
JP4256985,
Pamphlet of WO2007/086532,
JP-A-2009-120536,
JP-A-2009-67754,

JP-A-2009-67680,
JP-A-2009-57300,
JP4195615,
JP4158881,
JP4157245,
JP4157239,
JP4157227,
JP4118458,
JP-A-2008-74832,
JP3982770,
JP3801386,
Pamphlet of WO2005/028465,
Pamphlet of WO2003/042203,
JP-A-2005-289812,
JP-A-2005-289807,
JP-A-2005-112772,
JP3522189,
Pamphlet of WO2002/090342,
JP3471073,
JP-A-2003-277381,
Pamphlet of WO2001/060811
WO2000/071544,
Pamphlet of WO2005/028465,
Pamphlet of WO2011/16582, and
Pamphlet of WO2011/034202.

Among the above photochromic compounds, it is more desired to use one or more kinds of the chromene compounds having an indenonaphtho[2,1-f]naphtho[1,2-b] pyran skeleton from the standpoint of photochromic properties such as color density, initial color, durability and fading rate. Among these chromene compounds, further, the compounds having molecular weights of not less than 540 are desired because of their particularly excellent color densities and fading rates. Further, when the chromene compound having the indenonaphtho[2,1-f]naphtho[1,2-b]pyran skeleton is used, effects of the use of the component (A) are exhibited to a conspicuous degree. Shown below are concrete examples of the chromene compound having the indenonaphtho[2,1-f]naphtho[1,2-b]pyran skeleton.

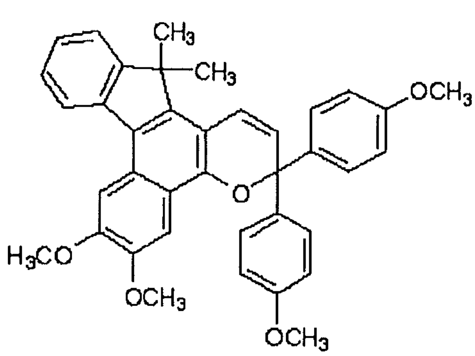

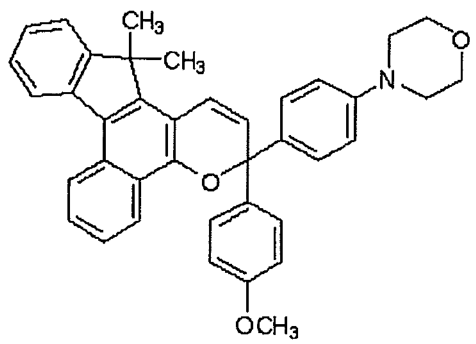

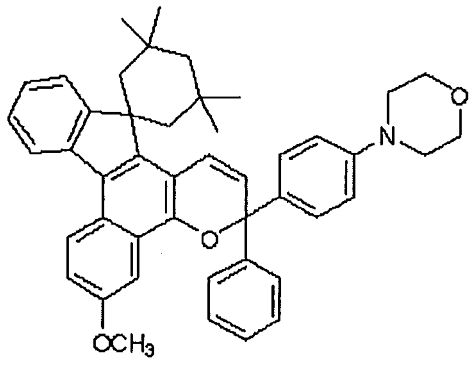

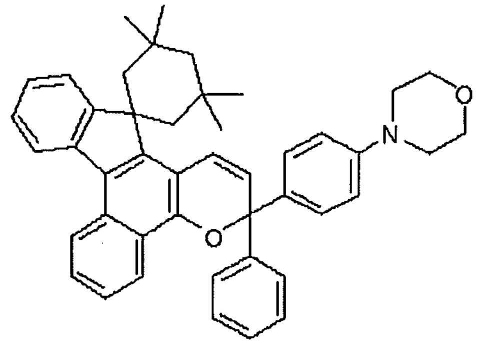

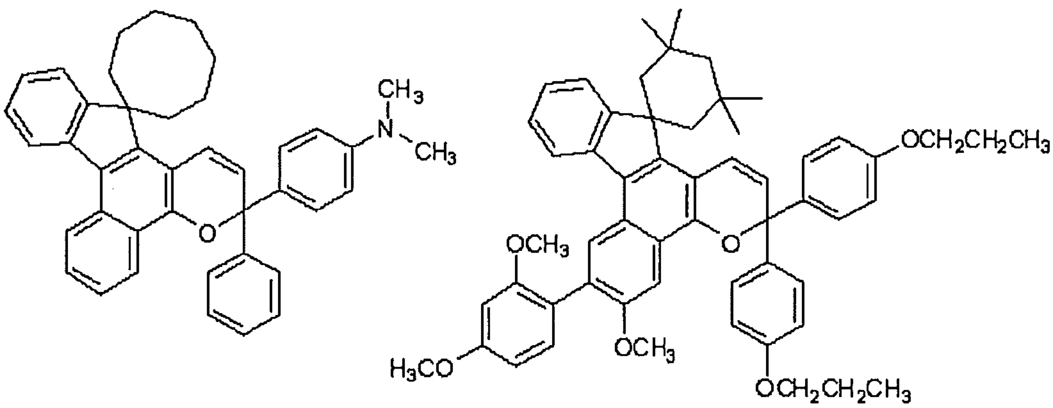

<Ratio of adding the photochromic curable composition>

In the invention, the amounts of adding the components may be suitably determined depending on the use of the cured body that is obtained. In order for the obtained cured body to exhibit excellent photochromic properties and durability, however, it is desired that the following amounts of addition are satisfied.

Namely, if the component (B) is presumed to be 100 parts by mass, it is desired that the component (A) is 0.01 to 20 parts by mass and the component (C) is 0.01 to 20 parts by mass.

With the component (A) satisfying the above range (0.01 to 20 parts by mass), the obtained cured body exhibits improved durability, suitable degree of hardness, and sufficiently large strength and heat resistance.

With the component (C) satisfying the above range (0.01 to 20 parts by mass), further, the obtained cured body possesses excellent photochromic properties.

When the photochromic curable composition of the invention is to be used for the photochromic spectacle lenses, it is specifically desired that the photochromic curable composition is added in amounts as described below depending on the in-mass method and the coating method that are described above.

That is, when the in-mass method is employed and when the component (B) is presumed to be 100 parts by mass, it is desired that the component (A) is 0.01 to 5 parts by mass, preferably, 0.02 to 3 parts by mass and, specifically, 0.05 to 1 part by mass. It is, further, desired that the component (C) is 0.01 to 5 parts by mass, preferably, 0.02 to 3 parts by mass and, specifically, 0.05 to 1 part by mass.

On the other hand, when the coating method is employed and when the component (B) is presumed to be 100 parts by mass, it is desired that the component (A) is 0.1 to 20 parts by mass, preferably, 0.5 to 10 parts by mass and, specifically, 1 to 5 parts by mass. It is, further, desired that the component (C) is 0.1 to 20 parts by mass, preferably, 0.5 to 10 parts by mass and, specifically, 1 to 5 parts by mass.

Desired ratios of addition are as described above when the photochromic cured body (lens) is to be produced by the in-mass method or the coating method. Here, it is desired that the mass ratio of the component (A) and the component (C) {mass ratio: component (A)/component (C)}, is 0.1 to 10 and, specifically, 0.5 to 2.

<Other components>

Next, described below are other components that can be added to the photochromic curable composition of

the present invention.

In order to improve repeat durability of the photochromic compound, color-developing rate, fading rate and moldability, the curable composition of the invention can be, further, blended with additives such as surfactant, antioxidant, radical-trapping agent, ultraviolet stabilizer, ultraviolet ray absorber, parting agent, anti-tinting agent, antistatic agent, fluorescent dye, dye, pigment, perfume and plasticizer. It is also allowable to add a polymerization initiator that will be described later to cure the curable composition. The polymerization initiator will be described later in detail in the method of producing the cured body. As the additives to be used, there can be used any known compounds without limitation.

The surfactant to be used may be any one of the nonionic type, anionic type or cationic type. From the standpoint of solubility in the polymerizable monomer, however, it is desired to use the nonionic surfactant. As the nonionic surfactant that can be preferably used, there can be exemplified sorbitan fatty acid ester, polyethylene glycol fatty acid ester and polyoxyethylene alkyl ether. The surfactants may be used in two or more kinds being mixed together. The amount of adding the surfactant is, desirably, in a range of 0.1 to 20 parts by mass per 100 parts by mass of the whole radically polymerizable monomers {total amount of the component (A) and the component (B)}.

As the antioxidant, radical-trapping agent, ultraviolet stabilizer and/or ultraviolet ray absorber, there can be preferably used hindered amine light stabilizer, hindered phenol antioxidant, phenol type radical-trapping agent, sulfur type antioxidant, benzotriazole type compound and benzophenone type compound. They may be used in two or more kinds being mixed together. Further, they may be used in combination with the surfactant. Amounts of addition of these antioxidant, radical-trapping agent, ultraviolet stabilizer and/or ultraviolet ray absorber are, desirably, in a range of 0.001 to 20 parts by mass per 100 parts by mass of the whole radically polymerizable monomers.

The above hindered amine light stabilizer can be added supplementary to a degree that does not impair the effect of the invention.

When the curable composition of the invention is used as a coating agent, further, there can be added, as other useful stabilizer, a hindered phenol antioxidant from the standpoint of improving the repeat durability of the cured body. As the hindered phenol antioxidant, there can be used any known compound without limitation. From the standpoint of preventing the deterioration of the photochromic compound, however, it is desired to use, specifically, IRGANOX 245 produced by Chiba Specialty Chemicals Co.:

Ethylenebis(oxyethylene)bis[3,5-tert-butyl-4-hydroxy-m-toluyl] propionate,

IRGANOX 1076 produced by Chiba Specialty Chemicals Co.:

Octadecyl-3-(3,5-di-tert-butyl-4-hydroxyphenyl) propionate,

IRGANOX 1010 produced by Chiba Specialty Chemicals Co.:

Pentaerythritoltetrakis[3-(3,5-di-tert-butyl-4-hydroxyphenyl) propionate].

The hindered phenol antioxidant may be added in an amount in a range of 0.001 to 20 parts by mass, preferably, in a range of 0.1 to 10 parts by mass and, more preferably, in a range of 1 to 10 parts by mass per 100 parts by mass of the whole radically polymerizable monomers.

<Method of preparing the photochromic curable composition, method of forming the cured body thereof and use thereof>

Next, described below are the method of preparing the photochromic curable composition, method of forming the cured body thereof and use thereof.

There is no specific limitation on the preparation of the photochromic curable composition of the invention, and the components may be weighed by predetermined amounts and may be mixed together. There is no specific limitation on the order of adding the components; i.e., all of the components may be added together simultaneously or the polymerizable monomer components only may be mixed together in advance and, thereafter, the photochromic compounds and other additives may be added just prior to the polymerization as will be described later. Further, as will be described later, a polymerization initiator, too, can be added, as required, for the polymerization.

It is desired that the photochromic curable composition of the present invention has a viscosity at 25°C of 5 to 500 cPs. When used as a coating agent, the viscosity is, preferably, 20 to 500 cPs, more preferably, 50 to 300 cPs and, specifically preferably, 60 to 200cPs. With the viscosity lying in this range, it is allowed to easily form the coating in a thickness which is slightly as large as 10 to 100 $\mu$m and to obtain the photochromic properties to a sufficient degree. When used as the in-mass type, its viscosity at 25°C is, preferably, 5 to 300 cPs and, more preferably, 10 to 100 cPs. With the viscosity lying in this range, the operation for pouring the composition into the lens mold can be facilitated. The viscosity may be adjusted depending on the addition and/or the kind of the component (B) that is used.

There is no specific limitation on the method of obtaining the photochromic cured body by curing the photochromic curable composition of the present invention, and any known polymerization may be employed depending on the kinds of the polymerizable monomers that are used. The polymerization can be started by using various peroxides or radical polymerization initiators such as azo compounds, by the irradiation with ultraviolet rays, $\alpha$-rays, $\beta$-rays or $\gamma$-rays, or by using both of them.

As the radical polymerization initiator, there can be used any known compound without particular limitation. Described below are representative examples of the thermal polymerization initiator.

Diacyl peroxides such as benzoyl peroxide, p-chlorobenzoyl peroxide, decanoyl peroxide, lauroyl peroxide and acetyl peroxide;

Peroxy esters such as t-butylperoxy-2-ethyl hexanoate, t-butylperoxy dicarbonate, cumylperoxy neodecanoate and t-butylperoxy benzoate;

Percarbonates such as diisopropylperoxy dicarbonate, di-2-ethylhexylperoxy dicarbonate and di-sec-butyl peroxy dicarbonate; and

Azo compounds such as 2,2'-azobisisobutylonitrile, 2,2'-azobis(4-dimethylvaleronitrile), 2,2'-azobis (2-methylbutylonitrile) and 1,1'-azobis(cyclohexane-1-carbonitrile).

The amount of the thermal polymerization initiator that is to be used varies depending on the polymerization conditions, kind of the initiator, and the kinds of compositions of the polymerizable monomers, and cannot be exclusively determined. Usually, however, the thermal polymerization initiator is used in an amount in a range of 0.01 to 10 parts by mass per 100 parts by mass of the whole radically polymerizable monomers. The thermal polymerization initiator may be used in a single kind or may be used in a plurality of kinds being mixed together.

When the polymerization is to be conducted by the irradiation with light such as ultraviolet rays, further, the following compounds are desirably used as the photo polymerization initiator.

Benzoin,
Benzoinmethyl ether,
Benzoinbutyl ether,
Benzophenone,
Acetophenon 4,4'-dichlorobenzophenone,
Diethoxyacetophenone,
2-Hydroxy-2-methyl-1-phenylpropane-1-one,
Benzylmethylketal,
1-(4-Isopropylphenyl)-2-hydroxy-2-methylpropane-1-one,
1-Hydroxycyclohexylphenylketone,
2-Isopropylthioxanthone,
Bis(2,6-dimethoxybenzoyl-2,4,4,-trimethyl-pentylphosphinoxide,
Bis(2,4,6-trimethylbenzoyl)-phenylphosphonoxide, 2,4,6-Trimethylbenzoyldiphenyl-phosphinoxide, and 2-Benzyl-2-dimethylamino-l-(4-morpholinophenyl)-butanone-1.

These photo polymerization initiators are, usually, used in an amount in a range of 0.001 to 5 parts by mass per 100 parts by mass of the whole radically polymerizable monomers. The photo polymerization initiators may be used alone or in a plurality of kinds being mixed together. It is also allowable to use the above thermal polymerization initiator in combination with the photo polymerization initiator.

A particularly preferred method of obtaining a cured body from the photochromic curable composition of the invention comprises irradiating the photochromic curable composition of the invention containing the photo polymerization initiator with ultraviolet rays to cure it and, as required, followed by heating to complete the polymerization.

When the polymerization is to be conducted by the irradiation with ultraviolet rays, a known source of light can be used without any limitation. As the source of light, there can be used ultra-high pressure mercury lamp, high pressure mercury lamp, low pressure mercury lamp, xenon lamp, carbon arc, sterilization lamp, metal halide lamp or electrodeless lamp. The time for irradiation by using the above source of light may be suitably determined depending on the kind of the photo polymerization initiator, absorption wavelength and sensitivity, and the thickness of the photochromic layer. When an electron ray is used as the source of light, further, the photochromic layer can be cured without adding the photo polymerization initiator.

The thus obtained cured body can be used directly as a photochromic optical material. Usually, however, the cured body is coated with a hard coating to prevent the cured body from being scratched when it is used. The scratch resistance can thus be improved.

Any known coating agent (hard coating agent) can be used without limitation for forming the hard coating. Concretely, there can be used a hard coating agent comprising chiefly a silane coupling agent or a sol of an oxide such as of silicon, zirconium, antimony, aluminum or titanium, or a hard coating agent comprising chiefly an organic high

molecular material.

The cured body of the photochromic curable composition alone of the invention, the optical material obtained by forming a coating of the curable composition (coating agent) on the surfaces of the optical material, or the optical article obtained by, further, forming a hard coating on the above coating, can be, further, subjected to the working such as antireflection treatment, antistatic treatment and to the secondary treatment by, for example, vacuum-evaporating a thin film of a metal oxide such as $SiO_2$, $TiO_2$ or $ZrO_2$ or by applying a thin film of an organic high molecular material thereon.

EXAMPLES

The invention will now be described in further detail by way of Examples to which only, however, the invention is in no way limited.

<Synthesis of the component (A)>

(Synthesis Example 1)

Synthesis of a 2-acryloyloxyethylsuccinic acid (1,2,2,6,6-pentamethyl-4-piperidyl) ester (abbreviated as HM-01):

In a 500-ml four neck distillation flask, there were set stirrer vanes, a thermometer and a dropping funnel, and there were fed:

2-acryloyloxyethylsuccinic acid, 10.8 g (0.05 mols), 1,2,2,6,6-pentamethyl-4-hydroxypiperidine, 17.0 g (0.1 mol), 4-(N,N-dimethylamino)pyridine, 6.1 g (0.05 mols), and dehydrated tetrahydrofurane, 200 mL.

The mixture was cooled to 0°C and to which 12.1 g (0.012 mols) of a dicyclohexylcarbodiimide was added little by little. The mixture was stirred at 0 to 5°C for 10 minutes and was, further, stirred at room temperature overnight. The white solid matter that has precipitated was separated by filtration. To the filtrate was added 400 mL of toluene followed by washing with water three times each time with 400 mL of water. Further, the organic layer was washed two times each with 200 mL of 0.5N hydrochloric acid solution. The washing solution was collected, neutralized with a 5% sodium carbonate aqueous solution, and was extracted with toluene. After dried on magnesium sulfate, the solvent was distilled off. The obtained faintly yellow liquid was refined with a neutral alumina column {developing solution: chloroform/ethyl acetate = 3/1 (v/v)} to obtain 14.4 g of a colorless transparent liquid.

The product was elementally analyzed to be comprised of C61.54%, H8.42% and N3.91%. These values were in very good agreement with the values C61.77%, H8.46% and N3.79% calculated from $C_{19}H_{31}NO_6$.

Further, the nuclear magnetic resonance spectrum of protons indicated peaks of 27 protons at 1 to 5 ppm, peaks of 3 protons based on the acrylic group and a peak of a proton based on the hydrogen atom at the fourth position of the piperidyl group at 5 to 7 ppm.

From the above, it was confirmed that the colorless transparent liquid was a 2-acryloyloxyethylsuccinic acid (1,2,2,6,6-pentamethyl-4-piperidyl) ester represented by the following formula,

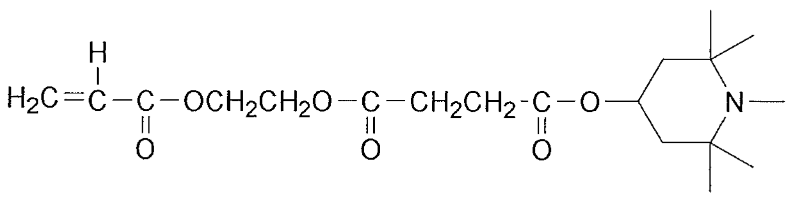

The yield thereof was 78%.

(Synthesis Example 2)

Synthesis of an acryloyloxypolycaprolactone (c ≒ 2) carboxylic acid (1,2,2,6,6-pentamethyl-4-piperidyl) ester (abbreviated as HM-02):

In the 500-ml four neck distillation flask, there were set the stirrer vanes, the thermometer and the dropping

funnel, and there were fed:

acryloyloxypolycaprolactone (c ≒ 2) carboxylic acid, 15.0 g (0.05 mols),
1,2,2,6,6-pentamethyl-4-hydroxypiperidine, 17.0 g (0.1 mol),
4-(N,N-dimethylamino)pyridine, 6.1 g (0.05 mols), and dehydrated tetrahydrofurane, 200 mL.

The mixture was cooled to 0°C and to which 12.1 g (0.012 mols) of the dicyclohexylcarbodiimide was added little by little. The mixture was stirred at 0 to 5°C for 10 minutes and was, further, stirred at room temperature overnight. The white solid matter that has precipitated was separated by filtration. To the filtrate was added 400 mL of toluene followed by washing with water three times each time with 400 mL of water. Further, the organic layer was washed two times each with 200 mL of 0.5N hydrochloric acid solution. The washing solution was collected, neutralized with the 5% sodium carbonate aqueous solution, and was extracted with toluene. After dried on magnesium sulfate, the solvent was distilled off. The obtained faintly yellow liquid was refined with the neutral alumina column {developing solution: chloroform/ethyl acetate = 3/1 (v/v)} to obtain 16.3 g of a colorless transparent liquid.

The product was elementally analyzed to be comprised of C66.02%, H9.39% and N3.18%. These values were in very good agreement with the values C66. 19%, H9. 55% and N3. 09% calculated from $C_{25}H_{43}NO_6$.

Further, the nuclear magnetic resonance spectrum of protons indicated peaks of 39 protons at 1 to 5 ppm, peaks of 3 protons based on the acrylic group and a peak of a proton based on the hydrogen atom at the fourth position of the piperidyl group at 5 to 7 ppm.

From the above, it was confirmed that the colorless transparent liquid was an acryloyloxypolycaprolactone (c ≒ 2) carboxylic acid (1,2,2,6,6-pentamethyl-4-piperidyl) ester represented by the following formula,

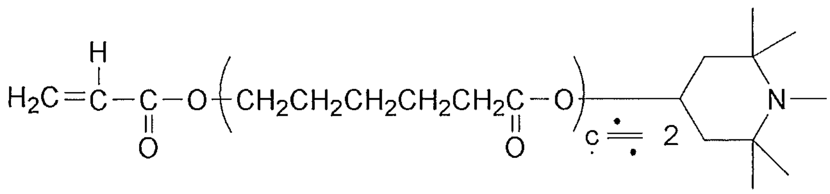

The yield thereof was 72%.

(Synthesis Example 3)

Synthesis of a 2-acryloyloxyethylsuccinic acid (1,2,2,6,6-pentamethyl-4-piperidyl) amide (abbreviated as HM-03):

In the 500-ml four neck distillation flask, there were set the stirrer vanes, the thermometer and the dropping funnel, and there were fed:

2-acryloyloxyethylsuccinic acid, 10.8 g (0.05 mols),
1,2,2,6,6-pentamethyl-4-aminopiperidine, 17.0 g (0.1 mol),
4-(N,N-dimethylamino)pyridine, 6.1 g (0.05 mols), and dehydrated tetrahydrofurane, 200 mL.

The mixture was cooled to 0°C and to which 12.1 g (0.012 mols) of the dicyclohexylcarbodiimide was added little by little. The mixture was stirred at 0 to 5°C for 10 minutes and was, further, stirred at room temperature overnight. The white solid matter that has precipitated was separated by filtration. To the filtrate was added 400 mL of toluene followed by washing with water three times each time with 400 mL of water. Further, the organic layer was washed two times each with 200 mL of 0.5N hydrochloric acid solution. The washing solution was collected, neutralized with the 5% sodium carbonate aqueous solution, and was extracted with toluene. After dried on magnesium sulfate, the solvent was distilled off. The obtained faintly yellow liquid was refined with the neutral alumina column {developing solution: chloroform/ethyl acetate = 3/1 (v/v)} to obtain 12.9 g of a colorless transparent liquid.

The product was elementally analyzed to be comprised of C61.80%, H8.65% and N7.73%. These values were in very good agreement with the values C61. 93%, H8.75% and N7.60% calculated from $C_{19}H_{32}N_2O_5$.

Further, the nuclear magnetic resonance spectrum of protons indicated peaks of 27 protons at 1 to 5 ppm, peaks of 3 protons based on the acrylic group, a peak of a proton based on the hydrogen atom at the fourth position of the piperidyl group and a peak of a proton based on amide group at 5 to 7 ppm.

From the above, it was confirmed that the colorless transparent liquid was a 2-acryloyloxyethylsuccinic acid (1,2,2,6,6-pentamethyl-4-piperidyl) amide represented by the following formula,

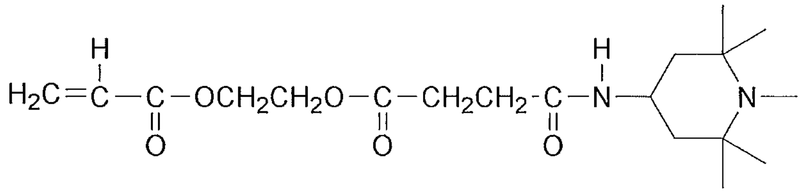

The yield thereof was 70%.

(Synthesis Example 4)

A 1,2,2,6,6-pentamethyl-4-piperidyl-polyethoxy (d ≒ 6) acrylate (HM-04) was obtained according to the description of JP-A-2001-71640. The compound HM-04 possessed the following structural formula,

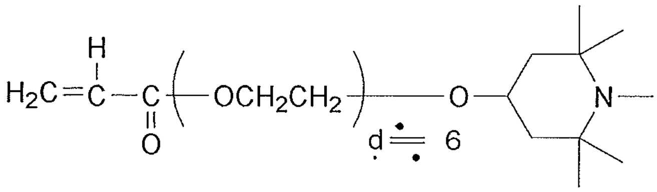

The nuclear magnetic resonance spectrum of protons of the product indicated peaks of 43 protons at 1 to 5 ppm, peaks of 3 protons based on the acrylic group and a peak of a proton based on the hydrogen atom at the fourth position of the piperidyl group at 5 to 7 ppm.

<Names of the components and abbreviations>

Described below are the abbreviations and names of the compounds that are used as the components (A), (B), (C) and other components.

Components (A):

HM-01: 2-acryloyloxyethylsuccinic acid (1,2,2,6,6-pentamethyl-4-piperidyl) ester
HM-02: acryloyloxypolycaprolactone (c ≒ 2) carboxylic acid (1,2,2,6,6-pentamethyl-4-piperidyl) ester
HM-03: 2-acryloyloxyethylsuccinic acid (1,2,2,6,6-pentamethyl-4-piperidyl) amide
HM-04: 1,2,2,6,6-pentamethyl-4-piperidyl-polyethoxy (d ≒ 6) acrylate.

Components (B):

Monofunctional (meth)acrylates:

GMA: glycidyl methacrylate
M90G: methoxypolyethylene glycol methacrylate
(average length of ethylene glycol chain, 9;
average molecular weight, 468)

Bifunctional (meth)acrylates:

BPE100: 2,2-bis[4-methacryloxy(polyethoxy)phenyl] propane (average length of ethylene glycol chain, 2.6)
BPE500: 2,2-bis[4-methacryloxy(polyethoxy)phenyl] propane (average length of ethylene glycol chain, 10)
4G: tetraethylene glycol dimethacrylate
A400: polyethylene glycol diacrylate (average length of ethylene glycol chain, 9; average molecular weight, 508)
UA-1: urethane diacrylate obtained by reacting a 2,2,4-trimethylhexamethylene diisocyanate with a hydroxyethyl acrylate at a mol ratio of 1:2
14G: polyethylene glycol dimethacrylate (average length of ethylene glycol chain, 14; average molecular

weight, 736)

Trifunctional or more highly functional (meth)acrylates:

TMPT: trimethylolpropane trimethacrylate
MA0735: polymethacryloxypropylpolyorganosiloxane compound (a mixture of cage-like silsesquioxanes having 8, 10 and 12 methacrylic groups in the molecules) (produced by Hybrid Plastics Co.)
Vinyl or allyl compounds:

αMS: α-methylstyrene
MSD: α-methylstyrene dimer

Component (C):

PC1:

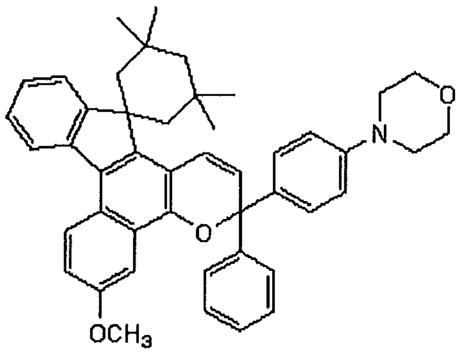

PC1

Other components:

Photo polymerization initiator:

CG1 1800: a mixture of 1-hydroxycyclohexylphenylketone and bis(2,6-dimethoxybenzoyl-2,4,4-trimethyl-pentylphosphinoxide (mass ratio, 3:1)
Thermal polymerization initiators:

Perbutyl ND: t-butylperoxyneodecanoate
Perocta O: 1,1,3,3-tetramethylbutylperoxy-2-ethyl hexanoate

Hindered amine light stabilizers:

LS765: sebacic acid (1,2,2,6,6-pentamethyl-4-piperidyl) diester
LA82: Methacrylic acid (1,2,2,6,6-pentamethyl-4-piperidyl) ester

Hindered phenol antioxidants:

IRGANOX245: ethylenebis(oxyethylene)bis[3,5-tert-butyl-4-hydroxy-m-toluyl] propionate
Moisture-curing primer: TR-SC-P manufactured by Tokuyama Co.

<Preparation of the photochromic curable compositions, production of the cured bodies and evaluation thereof>

(Example 1)

The following compounds; i.e.,

HM-01 prepared in Synthesis Example 1

(continued)

| | |
|---|---|
| | 0.5 parts by mass, |
| Components (B) | 100 parts by mass, |

| | |
|---|---|
| GMA | 1 part by mass, |
| BPE100 | 50 parts by mass, |
| 4G | 25 parts by mass, |
| A400 | 5 parts by mass, |
| TMPT | 10 parts by mass, |
| $\alpha$MS | 8 parts by mass, and |
| MSD | 1 part by mass, |
| Photochromic compound (PC1) | 0.05 parts by mass, |
| Polymerization initiator (perbutyl ND) | 1 part by mass, and |
| Polymerization initiator (perocta O) | 0.1 part by mass, |

were mixed together to a sufficient degree to obtain a photochromic curable composition. Table 1 shows the adding ratios of the photochromic curable composition. The photochromic curable composition (mixed solution) was poured into a mold constituted by a glass plate and a gasket of an ethylene-vinyl acetate copolymer to polymerize the photochromic curable composition by the cast polymerization. The polymerization was conducted by using an air oven, gradually elevating the temperature from 30°C up to 90°C over a period of 18 hours, and maintaining the temperature at 90°C for 2 hours. After the polymerization, the cured body was taken out from the glass mold. The obtained photochromic cured body (2 mm thick) was evaluated as described below to obtain the results as shown in Table 2.

(1) Color density.

The obtained photochromic cured body was irradiated with light from a xenon lamp {L-2480 (300W) SHL-100, manufactured by Hamamatsu Photonics Co.} through an aeromass filter (manufactured by Corning Co.) with a beam intensity of 365 = 2.4 mW/cm$^2$ and 245 nm = 24 $\mu$W/cm$^2$ on the surface of the photochromic coating for 300 seconds at 20°C $\pm$ 1°C to develop color. A maximum absorption wavelength at this moment was found by using a spectrophotometer {instantaneous multi-channel photo detector MCPD1000, manufactured by Otsuka Denshi Kogyo Co.}.

From the measured results, the color density was calculated according to the following formula.

$$\text{Color density} = \varepsilon(300) - \varepsilon(0)$$

wherein,

$\varepsilon$ (300) is an absorbency at the maximum absorption wavelength after irradiated with light for 300 seconds, and
$\varepsilon$ (0) is an absorbency of the cured body at the above wavelength in a state of not irradiated with light.

The higher the value is the more excellent the photochromic properties are.

(2) Fading half-life.

After the photochromic cured body was irradiated with light for 300 seconds, irradiation of light was discontinued, and the time {t1/2 (min) } was measured until the absorbency of the cured body at the maximum wavelength decreased down to one-half the value of { $\varepsilon$ (300) - $\varepsilon$ (0)}.

The shorter the time is the faster the fading rate is and the more excellent the photochromic properties are.

(3) Repeat durability.

In order to evaluate the repeat durability of the color developed by the irradiation with light, the deterioration

acceleration test was carried out as described below.

By using a xenon weatherometer {X25 manufactured by Suga Shikenki Co.}, the obtained photochromic cured body was deteriorated in an accelerated manner for 200 hours. The color density was evaluated before and after the deterioration; i.e., the cured body was measured for its color density (Ao) before the test and was measured for its color density ($A_{200}$) after the test.

From the measured results, the remaining ratio that roughly represents the repeat durability was calculated.

$$\text{Remaining ratio (\%)} = \{(A_{200}/A_0) \times 100\}$$

wherein,

$A_0$ is a color density of before the test, and
$A_{200}$ is color density of after the test.

By using a color difference meter {SM-4 manufactured by Suga Shikenki Co.}, further, a difference (ΔYI) in the yellowness was found.

$$\Delta YI = YI_{200} - YI_0$$

wherein,

$YI_{200}$ is YI after deteriorated in an accelerated manner for 200 hours, and
$YI_0$ is YI before deteriorated.

The larger the remaining ratios are and the smaller the differences are in the yellowness, the larger the repeat durabilities are and more excellent the photochromic properties are. The evaluated results were as shown in Table 2.

(4) Turbidity.

In order to evaluate the turbidity on the surface when preserved for extended periods of time, the following preservation acceleration test was conducted. Namely, the obtained photochromic cured body was placed in a constant-temperature constant-humidity vessel maintained at 60°C and 98%RH for 72 hours. By using a hazeometer, a difference (ΔHAZE) in the value of haze before and after the test was found.

$$\Delta HAZE = HAZE_{72} - HAZE_0$$

wherein,

$HAZE_{72}$ is a HAZE of after 72 hours, and
$HAZE_0$ is a HAZE of before being put in the constant-temperature constant-humidity vessel.

(5) Peeling.

In the step of curing by polymerization, the components that do not take part in the polymerization separate away from the cured body giving rise to the occurrence of a phenomenon in that the cured body peels off from the glass mold. In this case, the surface of the lens comes in contact with the air causing the polymerization to be impaired, and the lens becomes defective. Ten pieces were polymerized and how many of them have peeled off was confirmed to find the rate of occurrence of peeling.

(Example 2)

The operation was carried out in the same manner as in Example 1 but using 0.5 parts by mass of HM-03 instead of 0.5 parts by mass of HM-01 and using 100 parts by mass of the components (B) comprised of,

| | |
|---|---|
| GMA | 1 part by mass, |
| M90G | 7 parts by mass, |
| 4G | 40 parts by mass, |
| A400 | 15 parts by mass, |
| UA-1 | 25 parts by mass, |
| TMPT | 10 parts by mass, |
| $\alpha$ MS | 1 part by mass, and |
| MSD | 1 part by mass, |

instead of using 100 parts by mass of the components (b) comprised of,

| | |
|---|---|
| GMA | 1 part by mass, |
| BPE100 | 50 parts by mass, |
| 4G | 25 parts by mass, |
| A400 | 5 parts by mass, |
| TMPT | 10 parts by mass, |
| $\alpha$ MS | 8 parts by mass, and |
| MSD | 1 part by mass. |

Table 1 shows the adding ratios of the photochromic curable composition and Table 2 shows the evaluated results.

(Comparative Example 1)

The operation was carried out in the same manner as in Example 1 but using 0.5 parts by mass of LS765 instead of using 0.5 parts by mass of HM-01. Table 1 shows the adding ratios of the photochromic curable composition and Table 2 shows the evaluated results.

The photochromic cured body prepared by using the above photochromic curable composition underwent peeling during the curing by polymerization.

Table 1

| No. | Component (A) | Component (B) | Component (C) | Others |
|---|---|---|---|---|
| Example 1 | HM-01 (0.5) | GMA/BPE100/4G/A400/TMPT/$\alpha$MS/MSD (1/50/25/5/10/8/1) | PC1 (0.05) | - |
| Example 2 | HM-03 (0.5) | GMA/M90G/4G/A400/UA-1/TMPT/$\alpha$MS/MSD (1/7/40/15/25/10/1/1) | PC1 (0.05) | - |
| Comparative Example 1 | - | GMA/BPE100/4G/A400/TMPT/$\alpha$MS/MSD (1/50/25/5/10/8/1) | PC1 (0.05) | LS765 (0.5) |

Table 2

| No. | Color density | Fading half-life (sec.) | Repeat durability | | Turbidity $\Delta$HAZE | Occurrence of peeling (%) |
|---|---|---|---|---|---|---|
| | | | Remaining ratio (%) | $\Delta$YI | | |
| Example 1 | 1.0 | 60 | 88 | 1.7 | 0 | 0 |
| Example 2 | 1.0 | 60 | 87 | 1.7 | 0 | 0 |
| Comparative Example 1 | 1.0 | 56 | 87 | 1.7 | 3 | 30 |

(Example 3)

As the photochromic compound, PC1 was added in an amount of 2 parts by weight to a mixture of:

| | |
|---|---|
| HM-02 prepared in Synthesis Example 2 | 5 parts by mass, and |
| Component (B) | 100 parts by mass, |
| comprised of: | |
| TMPT | 30 parts by mass, |
| BPE500 | 50 parts by mass, |
| A400 | 19 parts by mass, |
| and | |
| GMA | 1 part by mass, |

and was mixed therein to a sufficient degree. To the above mixture, there were added:

antioxidant (IRGANOX 245) 3 parts by mass, and
polymerization initiator (CGI 1800) 0.5 parts by mass.

The above compounds were mixed together to a sufficient degree to obtain a photochromic curable composition (coating agent). Table 3 shows the added ratios of the coating agent. By using a spin coater (1H-DX2 manufactured by MIKASA Co.), a moisture-curing primer TR-SC-P was applied onto the surface of a 2 mm-thick plastic lens (MR: thiourethane plastic lens; optical material having a reflective index = 1.60) at a revolving speed of 70 rpm for 15 seconds and, next, at 1000 rpm for another 10 seconds. Thereafter, the plastic lens was spin-coated with about 2 g of the coating agent obtained by the above method at a revolving speed of 60 rpm for 40 seconds and, further, at 600 rpm for 10 to 20 seconds so that the thickness of the photochromic coating was 40 $\mu$m. The lens having its surface coated was irradiated with light by using a metal halide lamp of an output of 200 mW/cm$^2$ in a nitrogen gas atmosphere for 90 seconds to cure the coating. Thereafter, the lens was heated at 110°C for one hour to produce an optical material having a photochromic coating.
The thus obtained optical material having the photochromic coating was evaluated in the same manner as in Example 1. The results were as shown in Table 4.

(Example 4)

The operation was carried out in the same manner as in Example 3 but using 5 parts by mass of HM-04 instead of using 5 parts by mass of HM-02. Table 3 shows the adding ratios of the photochromic curable composition and Table 4 shows the

evaluated results.

(Comparative Example 2)

The operation was carried out in the same manner as in Example 3 but using 5 parts by mass of LS765 instead of using 5 parts by mass of HM-02. Table 3 shows the adding ratios of the photochromic curable composition and Table 4 shows the

evaluated results.

The photochromic cured body prepared by using the above photochromic curable composition became turbid in the accelerated preservation test.

(Comparative Example 3)

The operation was carried out in the same manner as in Example 3 but using 5 parts by mass of LA-82 instead of using 5 parts by mass of HM-02. Table 3 shows the adding ratios of the photochromic curable composition and Table 4 shows the

evaluated results.

The photochromic cured body prepared by using the above photochromic curable composition had a long fading half-life and a slightly low durability.

Table 3

| No. | Component (A) | Component (B) | Component (C) | Others |
|---|---|---|---|---|
| Example 3 | HM-02 (5) | TMPT/BPE500/A400/GMA (30/50/19/1) | PC1 (2) | |
| Example 4 | HM-04 (5) | TMPT/BPE500/A400/GMA (30/50/19/1) | PC1 (2) | |
| Comparative Example 2 | - | TMPT/BPE500/A400/GMA (30/50/19/1) | PC1 (2) | LS765 (5) |
| Comparative Example 3 | - | TMPT/BPE500/A400/GMA (30/50/19/1) | PC1 (2) | LA82 (5) |

Table 4

| No. | Color density | Fading half-life (sec) | Repeat durability | | Turbidity ΔHAZE |
|---|---|---|---|---|---|
| | | | Remaining ratio (%) | ΔYI | |
| Example 3 | 1.0 | 53 | 82 | 1.9 | 0 |
| Example 4 | 1.0 | 60 | 80 | 1.9 | 0 |
| Comparative Example 2 | 1.0 | 50 | 82 | 1.9 | 18 |
| Comparative Example 3 | 0.9 | 92 | 76 | 3.0 | 0 |

(Synthesis Example 5)

Synthesis of a 2-acryloyloxypolyethoxy (a ≒ 2) succinic acid (1,2,2,6,6-pentamethyl-4-piperidyl) ester (abbreviated as HM-05).

In the 500-ml four neck distillation flask, there were set the stirrer vanes, the thermometer and the dropping funnel, and there were fed:

| | |
|---|---|
| succinic anhydride | 10.0 g (0.1 mol), |
| 1,2,2,6,6-pentamethyl-4-hydroxypiperidine, | 17.0 g (0.1 mol), |
| and | |
| dehydrated N,N-dimethylformamide, | 200 mL. |

The mixture was stirred at room temperature overnight and, thereafter, the N,N-dimethylformamide was distilled off under reduced pressure. To the obtained colorless transparent liquid was added 100 mL of toluene followed by stirring. The precipitated white solid matter was separated by filtration and was dried at room temperature in vacuum to obtain 25.0 g of a succinic acid mono(1,2,2,6,6-pentamethyl-4-piperidyl) ester (yield, 93%).

Next, in the 500-ml four neck distillation flask, there were set the stirrer vanes, the thermometer and the dropping funnel, and there were fed:

| | |
|---|---|
| succinic acid mono(1,2,2,6,6-pentamethyl-4-piperidyl) ester, | 13.5 g (0.05 mol), |
| polyethylene glycol (a ≒ 2) monoacrylate, | 8.0 g (0.05 mol), |
| 4-(N,N-dimethylamino) pyridine, | 6.1 g (0.05 mol), |
| and | |
| dehydrated tetrahydrofurane, | 200 mL. |

The mixture was cooled down to 0°C and to which 12.1 g (0.012 mols) of the dicyclohexylcarbodiimide was added little by little. The mixture was stirred at 0 to 5°C for 10 minutes and was, further, stirred at room temperature

overnight. The white solid matter that has precipitated was separated by filtration. Thereafter, to the filtrate was added 400 mL of toluene followed by washing three times with 400 mL of water each time. Further, the organic layer was washed two times each with 200 mL of 0.5N hydrochloric acid solution. The washing solution was collected, neutralized with the 5% sodium carbonate aqueous solution, and was extracted with toluene. After dried on magnesium sulfate, the solvent was distilled off. The obtained faintly yellow liquid was refined with the neutral alumina column {developing solution: chloroform/ethyl acetate = 3/1 (v/v) } to obtain 26.8 g of HM-05 as a colorless transparent liquid (yield, 65%).

The compound HM-05 possessed the following structural formula,

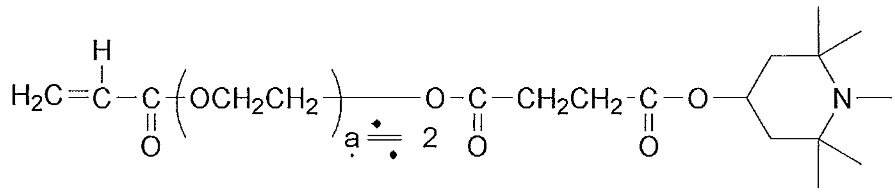

The nuclear magnetic resonance spectrum of protons of the HM-05 indicated peaks of 31 protons at 1 to 5 ppm, peaks of 3 protons based on the acrylic group and a peak of a proton based on the hydrogen atom at the fourth position of the piperidyl group at 5 to 7 ppm.

(Synthesis Example 6)

The operation was conducted in the same manner as in Synthesis Examples 1 to 3 and 5 to obtain a compound HM-06 which possessed the following structural formula,

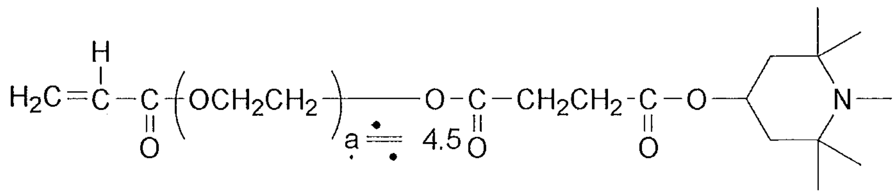

The nuclear magnetic resonance spectrum of protons of the HM-06 indicated peaks of 41 protons at 1 to 5 ppm, peaks of 3 protons based on the acrylic group and a peak of a proton based on the hydrogen atom at the fourth position of the piperidyl group at 5 to 7 ppm.

(Synthesis Example 7)

The operation was conducted in the same manner as in Synthesis Examples 1 to 3 and 5 to obtain a compound HM-07 which possessed the following structural formula,

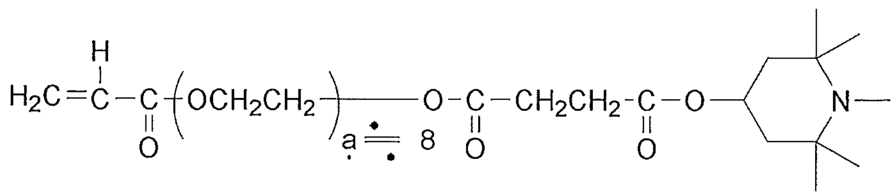

The nuclear magnetic resonance spectrum of protons of the HM-07 indicated peaks of 55 protons at 1 to 5 ppm, peaks of 3 protons based on the acrylic group and a peak of a proton based on the hydrogen atom at the fourth position of the piperidyl group at 5 to 7 ppm.

(Synthesis Example 8)

The operation was conducted in the same manner as in Synthesis Examples 1 to 3 and 5 to obtain a compound HM-08 which possessed the following structural formula,

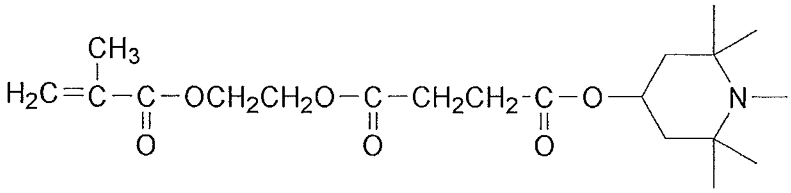

The nuclear magnetic resonance spectrum of protons of the HM-08 indicated peaks of 30 protons at 1 to 5 ppm, peaks of 2 protons based on the methacrylic group and a peak of a proton based on the hydrogen atom at the fourth position of the

piperidyl group at 5 to 7 ppm.

(Synthesis Example 9)

The operation was conducted in the same manner as in Synthesis Examples 1 to 3 and 5 to obtain a compound HM-09 which possessed the following structural formula,

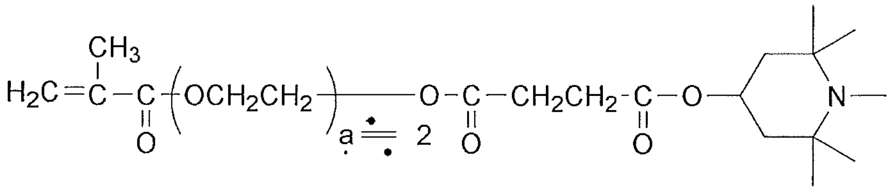

The nuclear magnetic resonance spectrum of protons of the HM-09 indicated peaks of 34 protons at 1 to 5 ppm, peaks of 2 protons based on the methacrylic group and a peak of a proton based on the hydrogen atom at the fourth position of the

piperidyl group at 5 to 7 ppm.

(Synthesis Example 10)

The operation was conducted in the same manner as in Synthesis Examples 1 to 3 and 5 to obtain a compound HM-10 which possessed the following structural formula,

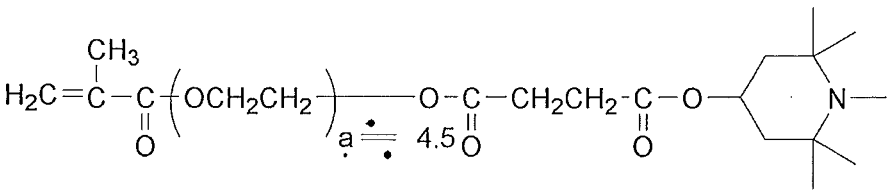

The nuclear magnetic resonance spectrum of protons of the HM-10 indicated peaks of 44 protons at 1 to 5 ppm, peaks of 2 protons based on the methacrylic group and a peak of a proton based on the hydrogen atom at the fourth position of the piperidyl group at 5 to 7 ppm.

(Synthesis Example 11)

The operation was conducted in the same manner as in Synthesis Examples 1 to 3 and 5 to obtain a compound HM-11 which possessed the following structural formula,

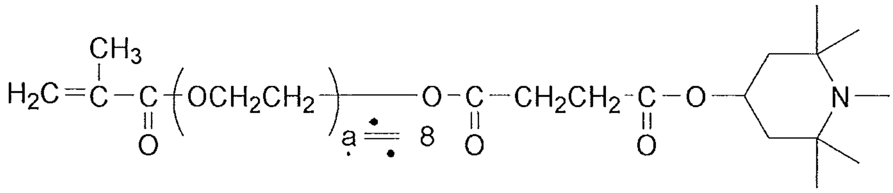

The nuclear magnetic resonance spectrum of protons of the HM-11 indicated peaks of 58 protons at 1 to 5 ppm, peaks of 2 protons based on the methacrylic group and a peak of a proton based on the hydrogen atom at the fourth position of the piperidyl group at 5 to 7 ppm.

(Synthesis Example 12)

The operation was conducted in the same manner as in Synthesis Examples 1 to 3 and 5 to obtain a compound HM-12 which possessed the following structural formula,

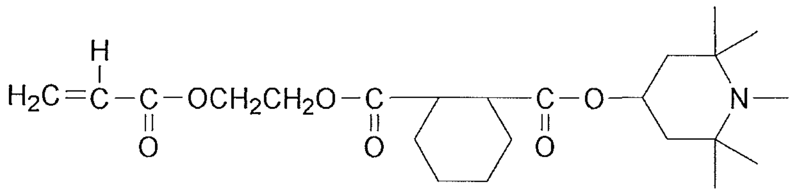

The nuclear magnetic resonance spectrum of protons of the HM-12 indicated peaks of 23 protons at 1 to 5 ppm, peaks of 3 protons based on the acrylic group and a peak of a proton based on the hydrogen atom at the fourth position of the piperidyl group at 5 to 7 ppm.

(Synthesis Example 13)

The operation was conducted in the same manner as in Synthesis Examples 1 to 3 and 5 to obtain a compound HM-13 which possessed the following structural formula,

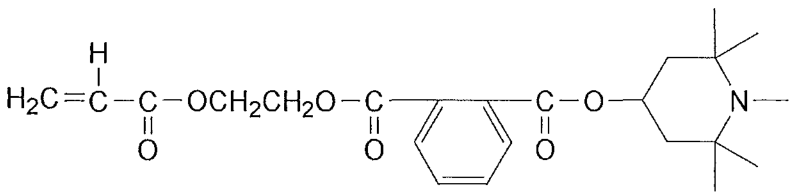

The nuclear magnetic resonance spectrum of protons of the HM-13 indicated peaks of 23 protons at 1 to 5 ppm, peaks of 3 protons based on the acrylic group, a peak of a proton based on the hydrogen atom at the fourth position of the piperidyl group and peaks of 4 protons based on the benzene ring at 5 to 9 ppm.

(Synthesis Example 14)

The operation was conducted in the same manner as in Synthesis Examples 1 to 3 and 5 to obtain a compound HM-14 which possessed the following structural formula,

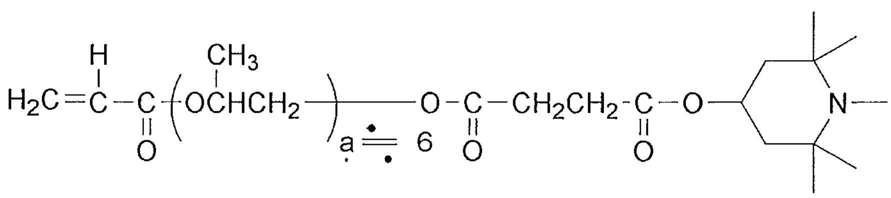

The nuclear magnetic resonance spectrum of protons of the HM-14 indicated peaks of 59 protons at 1 to 5 ppm, peaks of 3 protons based on the acrylic group and a peak of a proton based on the hydrogen atom at the fourth position of the piperidyl group at 5 to 7 ppm.

(Synthesis Example 15)

The operation was conducted in the same manner as in Synthesis Examples 1 to 3 and 5 to obtain a compound HM-15 which possessed the following structural formula,

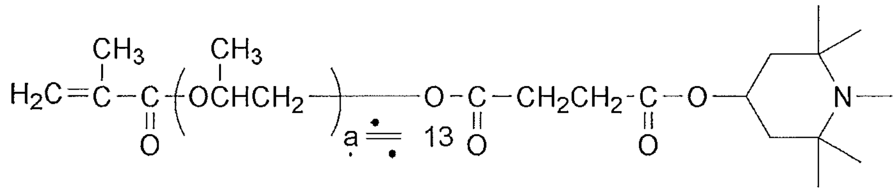

The nuclear magnetic resonance spectrum of protons of the HM-15 indicated peaks of 104 protons at 1 to 5 ppm, peaks of 2 protons based on the methacrylic group and a peak of a proton based on the hydrogen atom at the fourth position of the piperidyl group at 5 to 7 ppm.

(Synthesis Example 16)

The operation was conducted in the same manner as in Synthesis Examples 1 to 3 and 5 to obtain a compound HM-16 which possessed the following structural formula,

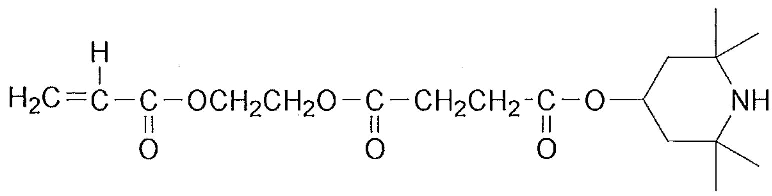

The nuclear magnetic resonance spectrum of protons of the HM-16 indicated peaks of 25 protons at 1 to 5 ppm, peaks of 3 protons based on the acrylic group and a peak of a proton based on the hydrogen atom at the fourth position of the piperidyl group at 5 to 7 ppm.

(Synthesis Example 17)

The operation was conducted in the same manner as in Synthesis Examples 1 to 3 and 5 to obtain a compound HM-17 which possessed the following structural formula,

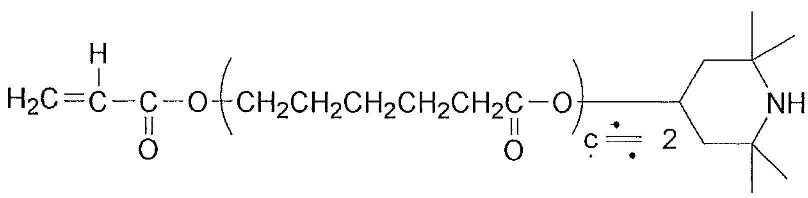

The nuclear magnetic resonance spectrum of protons of the HM-17 indicated peaks of 37 protons at 1 to 5 ppm, peaks of 3 protons based on the acrylic group and a peak of a proton based on the hydrogen atom at the fourth position of the piperidyl group at 5 to 7 ppm.

(Synthesis Example 18)

The operation was conducted in the same manner as in Synthesis Examples 1 to 3 and 5 to obtain a compound

HM-18 which possessed the following structural formula,

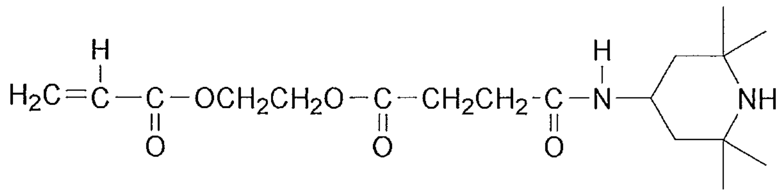

The nuclear magnetic resonance spectrum of protons of the HM-18 indicated peaks of 25 protons at 1 to 5 ppm, peaks of 3 protons based on the acrylic group, a peak of a proton based on the hydrogen atom at the fourth position of the piperidyl group and a peak of a proton based amide group at 5 to 7 ppm.

(Synthesis Example 19)

The operation was conducted in the same manner as in Synthesis Example 4 to obtain a compound HM-19 which possessed the following structural formula,

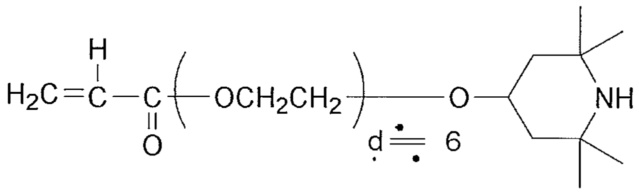

The nuclear magnetic resonance spectrum of protons of the HM-19 indicated peaks of 41 protons at 1 to 5 ppm, peaks of 3 protons based on the acrylic group and a peak of a proton based on the hydrogen atom at the fourth position of the piperidyl group at 5 to 7 ppm.

(Synthesis Example 20)

The operation was conducted in the same manner as in Synthesis Examples 1 to 3 and 5 to obtain a compound HM-20 which possessed the following structural formula,

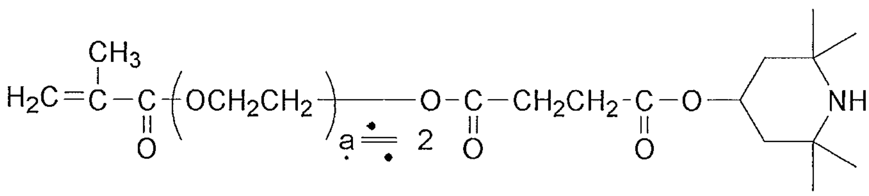

The nuclear magnetic resonance spectrum of protons of the HM-20 indicated peaks of 32 protons at 1 to 5 ppm, peaks of 2 protons based on the methacrylic group and a peak of a proton based on the hydrogen atom at the fourth position of the piperidyl group at 5 to 7 ppm.

(Synthesis Example 21)

The operation was conducted in the same manner as in Synthesis Examples 1 to 3 and 5 to obtain a compound HM-21 which possessed the following structural formula,

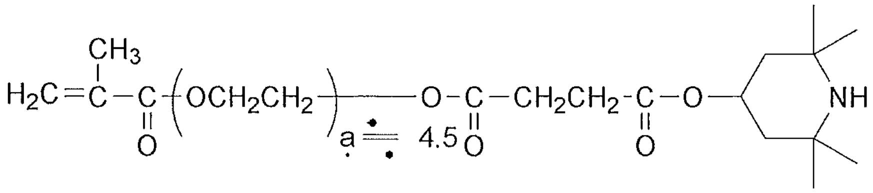

The nuclear magnetic resonance spectrum of protons of the HM-21 indicated peaks of 42 protons at 1 to 5 ppm, peaks of 2 protons based on the methacrylic group and a peak of a proton based on the hydrogen atom at the fourth position of the piperidyl group at 5 to 7 ppm.

(Examples 5 to 8)

The operation was conducted in the same manner as in Examples 1 and 2 to obtain photochromic cured bodies. Table 5 shows the adding ratios of the photochromic curable compositions and Table 6 shows the evaluated results.

Table 5

| No. | Component (A) | Component (B) | Component (C) | Others |
|---|---|---|---|---|
| Example 5 | HM-05 (0.5) | GMA/BPE100/4G/A400/TMPT/αMS/MSD (1/50/25/5/10/8/1) | PC1 (0.05) | - |
| Example 6 | HM-07 (1) | GMA/M90G/4G/A400/UA-1/TMPT/αMS/MSD (1/7/40/15/25/10/1/1) | PC1 (0.05) | - |
| Example 7 | HM-17 (0.5) | GMA/BPE100/4G/A400/TMPT/αMS/MSD (1/50/25/5/10/8/1) | PC1 (0.05) | - |
| Example 8 | HM-19 (0.5) | GMA/M90G/4G/A400/UA-1/TMPT/αMS/MSD (1/7/40/15/25/10/1/1) | PC1 (0.05) | - |

Table 6

| No. | Color density | Fading half-life (sec) | Repeat durability: Remaining ratio (%) | Repeat durability: ΔYI | Turbidity ΔHAZE | Occurrence of peeling (%) |
|---|---|---|---|---|---|---|
| Example 5 | 1.0 | 60 | 88 | 1.7 | 0 | 0 |
| Example 6 | 1.0 | 58 | 87 | 1.7 | 0 | 0 |
| Example 7 | 1.0 | 62 | 88 | 1.7 | 0 | 0 |
| Example 8 | 1.0 | 63 | 87 | 1.7 | 0 | 0 |

(Examples 9 to 21)

The operation was carried out in the same manner as in Example 3 to obtain photochromic cured bodies. Table 7 shows the adding ratios of the photochromic curable compositions and Table 8 shows the evaluated results.

Table 7

| No. | Component (A) | Component (B) | Component (C) | Others |
|---|---|---|---|---|
| Example 9 | HM-06 (5) | TMPT/BPE500/A400/GMA (30/50/19/1) | PC1 (2) | - |
| Example 10 | HM-08 (5) | MA0735/TMPT/BPE500/14G/GMA (40/15/15/29/1) | PC1 (2) | - |
| Example 11 | HM-09 (5) | TMPT/BPE500/A400/GMA (30/50/19/1) | PC1 (2) | - |
| Example 12 | HM-10 (5) | TMPT/BPE500/A400/GMA (30/50/19/1) | PC1 (2) | - |

(continued)

| No. | Component (A) | Component (B) | Component (C) | Others |
|---|---|---|---|---|
| Example 13 | HM-11 (10) | TMPT/BPE500/A400/GMA (30/50/19/1) | PC1 (2) | - |
| Example 14 | HM-12 (5) | TMPT/BPE500/A400/GMA (30/50/19/1) | PC1 (2) | - |
| Example 15 | HM-13 (5) | TMPT/BPE500/A400/GMA (30/50/19/1) | PC1 (2) | - |
| Example 16 | HM-14 (5) | TMPT/BPE500/A400/GMA (30/50/19/1) | PC1 (2) | - |
| Example 17 | HM-15 (5) | TMPT/BPE500/A400/GMA (30/50/19/1) | PC1 (2) | - |
| Example 18 | HM-16 (5) | TMPT/BPE500/A400/GMA (30/50/19/1) | PC1 (2) | - |
| Example 19 | HM-18 (5) | TMPT/BPE500/A400/GMA (30/50/19/1) | PC1 (2) | - |
| Example 20 | HM-20 (5) | MA0735/TMPT/BPE500/14G/GMA (40/15/15/29/1) | PC1 (2) | - |
| Example 21 | HM-21 (2) | TMPT/BPE500/A400/GMA (30/50/19/1) | PC1 (2) | - |

Table 8

| No. | Color density | Fading half-life (sec) | Repeat durability | | Turbidity ΔHAZE |
|---|---|---|---|---|---|
| | | | Remaining ratio (%) | ΔYI | |
| Example 9 | 1.0 | 50 | 83 | 1.9 | 0 |
| Example 10 | 1.0 | 48 | 86 | 1.9 | 0 |
| Example 11 | 1.0 | 50 | 86 | 1.9 | 0 |
| Example 12 | 1.0 | 50 | 86 | 1.9 | 0 |
| Example 13 | 1.0 | 50 | 83 | 2.1 | 0 |
| Example 14 | 1.0 | 53 | 84 | 1.9 | 0 |
| Example 15 | 0.9 | 55 | 84 | 2.0 | 0 |
| Example 16 | 1.0 | 50 | 82 | 2.2 | 0 |
| Example 17 | 1.0 | 50 | 79 | 2.4 | 0 |
| Example 18 | 1.0 | 54 | 84 | 2.0 | 0 |
| Example 19 | 1.0 | 54 | 84 | 2.0 | 0 |
| Example 20 | 1.0 | 47 | 86 | 1.9 | 0 |
| Example 21 | 1.0 | 51 | 82 | 2.3 | 0 |

## Claims

**1.** A photochromic curable composition that contains (A) a (meth)acrylate compound represented by the following formula (1), (B) a radically polymerizable monomer other than the (meth) acrylate compound represented by the formula (1), and (C) a photochromic compound,

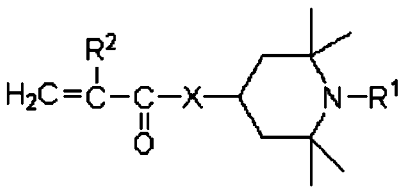

(1)

wherein,

$R^1$ and $R^2$ are, respectively, hydrogen atoms or methyl groups, and
X is a divalent group represented by the following formula (X1), (X2) or (X3),

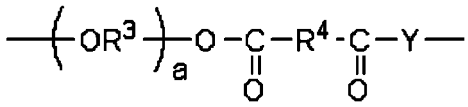 (X 1)

wherein,

$R^3$ is an alkylene group having 1 to 6 carbon atoms,
$R^4$ is an alkylene group having 1 to 6 carbon atoms, a cycloalkylene group having 3 to 8 carbon atoms and which may include a double bond, or an arylene group having 6 to 10 carbon atoms,
Y is an oxygen atom or a divalent group represented by -NH-, and
a is a positive number of 1 to 20,

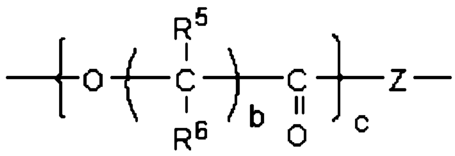 (X 2)

wherein,

$R^5$ and $R^6$ are, respectively, hydrogen atoms or methyl groups,
Z is an oxygen atom or a divalent group represented by -NH-,
b is a positive number of 2 to 7, and
c is a positive number of 1 to 20, or

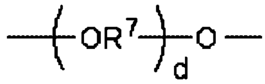 (X 3)

wherein,

$R^7$ is an alkylene group having 1 to 6 carbon atoms, and d is a positive number of 1 to 20.

2. The photochromic curable composition according to claim 1, which contains (A) the (meth)acrylate compound represented by said formula (1) in an amount of 0.01 to 20 parts by mass, (B) the radically polymerizable monomer other than the (meth) acrylate compound represented by said formula (1) in an amount of 100 parts by mass, and (C) the photochromic compound in an amount of 0.01 to 20 parts by mass.

3. The photochromic curable composition according to claim 1, wherein said photochromic compound (C) contains a compound having an indeno[2,1-f]naphtho[1,2-b]pyran skeleton.

4. A coating agent containing the photochromic curable composition of claim 1.

5. An optical material having a photochromic coating obtained by curing the coating agent of claim 4 on at least one surface of an optical base material.

6. A photochromic cured body obtained by curing the photochromic curable composition of claim 1.

7. A (meth)acrylate compound represented by the following formula (1-X1);

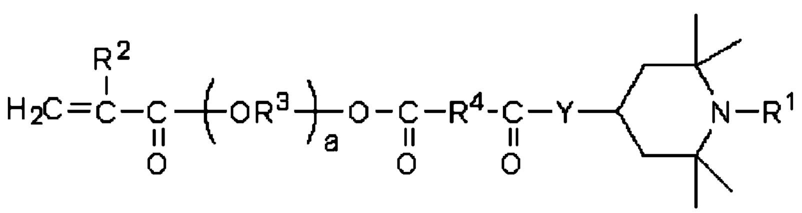 (1 - X 1)

wherein,

$R^1$ and $R^2$ are, respectively, hydrogen atoms or methyl groups,
$R^3$ is an alkylene group having 1 to 6 carbon atoms,
$R^4$ is an alkylene group having 1 to 6 carbon atoms, a cycloalkylene group having 3 to 8 carbon atoms and which may include a double bond, or an arylene group having 6 to 10 carbon atoms,
Y is an oxygen atom or a divalent group represented by -NH-, and
a is a positive number of 1 to 20.

**Patentansprüche**

1. Photochrome härtbare Zusammensetzung, die Folgendes enthält: (A) eine (Meth)acrylatverbindung derfolgenden Formel (1), (B) ein radikalisch polymerisierbares Monomer, das sich von der (Meth)acrylatverbindung der Formel (1) unterscheidet, und (C) eine photochrome Verbindung,

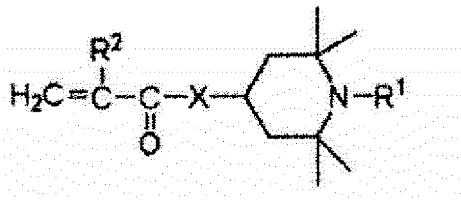
(1)

wobei

$R^1$ und $R^2$ jeweils Wasserstoffatome oder Methylgruppen bedeuten und
X eine zweiwertige Gruppe der folgenden Formeln (X1), (X2) oder (X3) bedeutet,

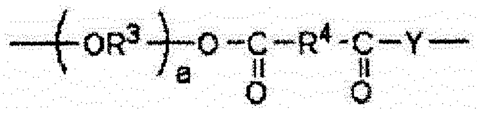
(X 1)

wobei

$R^3$ eine Alkylengruppe mit 1 bis 6 Kohlenstoffatomen bedeutet,
$R^4$ eine Alkylengruppe mit 1 bis 6 Kohlenstoffatomen, eine Cycloalkylengruppe mit 3 bis 8 Kohlenstoffatomen, die eine Doppelbindung enthalten kann, oder eine Arylengruppe mit 6 bis 10 Kohlenstoffatomen bedeutet,
Y ein Sauerstoffatom oder eine durch -NH- wiedergegebene zweiwertige Gruppe bedeutet, und
a eine positive Zahl von 1 bis 20 bedeutet,

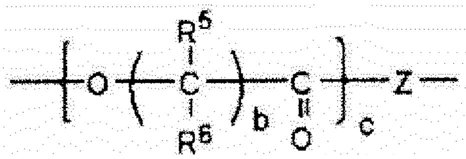
(X 2)

wobei

$R^5$ und $R^6$ jeweils Wasserstoffatome oder Methylgruppen bedeuten,
Z ein Sauerstoffatom oder eine durch -NH- wiedergegebene zweiwertige Gruppe bedeutet,
b eine positive Zahl von 2 bis 7 bedeutet und
c eine positive Zahl von 1 bis 20 bedeutet,
oder

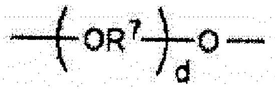 (X 3)

wobei

$R^7$ eine Alkylengruppe mit 1 bis 6 Kohlenstoffatomen bedeutet und
d eine positive Zahl von 1 bis 20 bedeutet.

**2.** Photochrome härtbare Zusammensetzung nach Anspruch 1, die Folgendes enthält: (A) die (Meth)acrylatverbindung der Formel (1) in einer Menge von 0,01 bis 20 Masseteilen, (B) das radikalisch polymerisierbare Monomer, das sich von der (Meth)acrylatverbindung der Formel (1) unterscheidet, in einer Menge von 100 Masseteilen und (C) die photochrome Verbindung in einer Menge von 0,01 bis 20 Masseteilen.

**3.** Photochrome härtbare Zusammensetzung nach Anspruch 1, wobei die photochrome Verbindung (C) eine Verbindung mit einem Indeno[2,1-f]naphtho[1,2-b]pyran-Gerüst enthält.

**4.** Beschichtungsmittel, enthaltend die photochrome härtbare Zusammensetzung nach Anspruch 1.

**5.** Optisches Material mit einer photochromen Beschichtung, erhalten durch Härten des Beschichtungsmittels von Anspruch 4 auf mindestens einer Oberfläche eines optischen Grundmaterials.

**6.** Photochromer gehärteter Körper, erhalten durch Härten der photochromen härtbaren Zusammensetzung nach Anspruch 1.

**7.** (Meth)acrylatverbindung der folgenden Formel (1-X1)

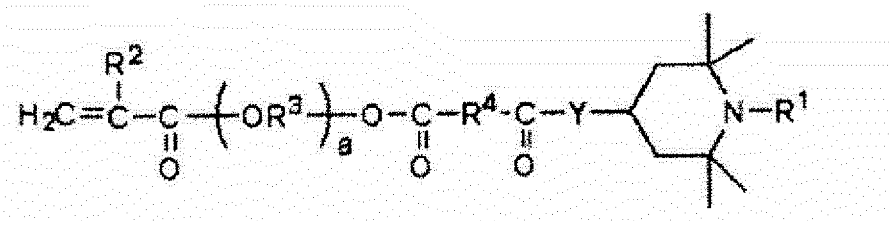 (1 - X 1)

wobei

$R^1$ und $R^2$ jeweils Wasserstoffatome oder Methylgruppen bedeuten,
$R^3$ eine Alkylengruppe mit 1 bis 6 Kohlenstoffatomen bedeutet,
$R^4$ eine Alkylengruppe mit 1 bis 6 Kohlenstoffatomen, eine Cycloalkylengruppe mit 3 bis 8 Kohlenstoffatomen, die eine Doppelbindung enthalten kann, oder eine Arylengruppe mit 6 bis 10 Kohlenstoffatomen bedeutet,
Y ein Sauerstoffatom oder eine durch -NH- wiedergegebene zweiwertige Gruppe bedeutet, und
a eine positive Zahl von 1 bis 20 bedeutet.

**Revendications**

**1.** Composition durcissable photochromique qui contient (A) un composé (méth)acrylate représenté par la formule (1) suivante, (B) un monomère radicalement polymérisable autre que le composé (méth)acrylate représenté par la formule (1), et (C) un composé photochromique,

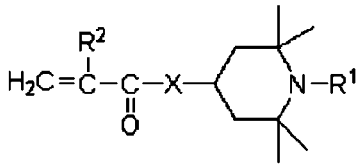

(1)

dans laquelle

$R^1$ et $R^2$ représentent, respectivement, des atomes d'hydrogène ou des groupes méthyle, et X est un groupe divalent représenté par la formule (X1), (X2) ou (X3) suivante

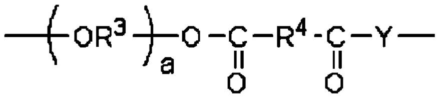

(X 1)

dans laquelle

$R^3$ représente un groupe alkylène ayant de 1 à 6 atomes de carbone,
$R^4$ représente un groupe alkylène ayant de 1 à 6 atomes de carbone, un groupe cycloalkylène ayant de 3 à 8 atomes de carbone et qui peut comporter une double liaison, ou un groupe arylène ayant de 6 à 10 atomes de carbone,
Y représente un atome d'oxygène ou un groupe divalent représenté par -NH-, et
a est un nombre positif de 1 à 20,

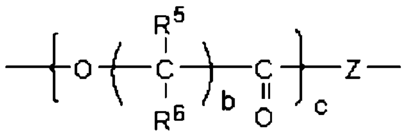

(X 2)

dans laquelle

$R^5$ et $R^6$ représentent, respectivement, des atomes d'hydrogène ou des groupes méthyle,
Z représente un atome d'oxygène ou un groupe divalent représenté par -NH-,
b est un nombre positif de 2 à 7, et
c est un nombre positif de 1 à 20, ou

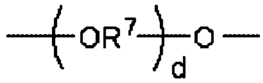

(X 3)

dans laquelle

$R^7$ représente un groupe alkylène ayant de 1 à 6 atomes de carbone, et
d est un nombre positif de 1 à 20.

**2.** Composition durcissable photochromique selon la revendication 1, qui contient (A) le composé (méth)acrylate représenté par ladite formule (1) dans une quantité de 0,01 à 20 parties en masse, (B) le monomère radicalement polymérisable autre que le composé (méth)acrylate représenté par ladite formule (1) dans une quantité de 100 parties en masse, et (C) le composé photochromique dans une quantité de 0,01 à 20 parties en masse.

**3.** Composition durcissable photochromique selon la revendication 1, dans laquelle ledit composé photochromique (C) contient un composé ayant un squelette indéno[2,1-f]naphto[1,2-b]pyrane.

**4.** Agent d'enrobage contenant la composition durcissable photochromique selon la revendication 1.

**5.** Matériau optique ayant un enrobage photochromique obtenu par durcissement de l'agent d'enrobage selon la revendication 4 sur au moins une surface d'un matériau optique de base.

**6.** Corps durci photochromique obtenu par durcissement de la composition durcissable photochromique selon la revendication 1.

**7.** Composé (méth)acrylate représenté par la formule (1-X1) suivante :

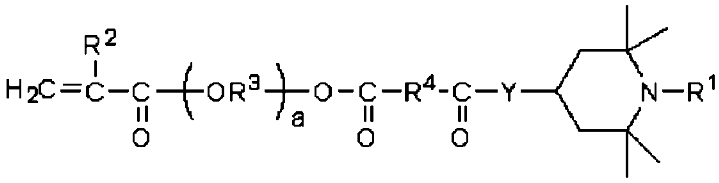

(1 - X 1)

dans laquelle

$R^1$ et $R^2$ représentent, respectivement, des atomes d'hydrogène ou des groupes méthyle,
$R^3$ représente un groupe alkylène ayant de 1 à 6 atomes de carbone,
$R^4$ représente un groupe alkylène ayant de 1 à 6 atomes de carbone, un groupe cycloalkylène ayant de 3 à 8 atomes de carbone et qui peut comporter une double liaison, ou un groupe arylène ayant de 6 à 10 atomes de carbone,
Y représente un atome d'oxygène ou un groupe divalent représenté par -NH-, et
a est un nombre positif de 1 à 20.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20015854 A
- WO 200311967 A
- JP 2001071640 A
- WO 2001005854 A
- WO 200483268 A
- WO 200975388 A
- JP 2028154 A
- JP 62288830 A
- WO 9422850 A
- WO 9614596 A
- JP 2001031670 A
- JP 2001011067 A
- JP 2001011066 A
- JP 2000344761 A
- JP 2000327675 A
- JP 2000256347 A
- JP 2000229976 A
- JP 2000229975 A
- JP 2000229974 A
- JP 2000229973 A
- JP 2000229972 A
- JP 2000219678 A
- JP 2000219686 A
- JP 11322739 A
- JP 11286484 A
- JP 11279171 A
- JP 9218301 A
- JP 9124645 A
- JP 8295690 A
- JP 8176139 A
- JP 8157467 A
- US P5645767 A
- US P5658501 A
- US 5961892 A
- US P6296785 B
- JP 4424981 B
- JP 4424962 B
- WO 2009136668 A
- WO 2008023828 A
- JP 4369754 B
- JP 4301621 B
- JP 4256985 B
- WO 2007086532 A
- JP 2009120536 A
- JP 2009067754 A
- JP 2009067680 A
- JP 2009057300 A
- JP 4195615 B
- JP 4158881 B
- JP 4157245 B
- JP 4157239 B
- JP 4157227 B
- JP 4118458 B
- JP 2008074832 A
- JP 3982770 B
- JP 3801386 B
- WO 2005028465 A
- WO 2003042203 A
- JP 2005289812 A
- JP 2005289807 A
- JP 2005112772 A
- JP 3522189 B
- WO 2002090342 A
- JP 3471073 B
- JP 2003277381 A
- WO 2001060811 A
- WO 2000071544 A
- WO 201116582 A
- WO 2011034202 A